# FASCICULE DE BREVET EUROPEEN

(11) **EP 3 065 733 B1**
(45) Date de publication et mention de la délivrance du brevet: **30.05.2018**
(21) Numéro de dépôt: 14790233.2
(22) Date de dépôt: 08.10.2014
(51) Int. Cl.: A61K 31/231, A23L 33/00, A61P 3/00, A61P 3/06, A61P 3/10, A61K 31/201, A61P 11/00, A61P 11/12

(54) **COMPOSES ET COMPOSITIONS COMPRENANT DE TELS COMPOSES POUR LA PRÉVENTION OU LE TRAITEMENT DES DYSLIPIDÉMIES**
VERBINDUNGEN UND ZUSAMMENSETZUNGEN MIT SOLCHEN VERBINDUNGEN ZUR VORBEUGUNG ODER BEHANDLUNG VON DYSLIPIDÄMIEN
COMPOUNDS AND COMPOSITIONS COMPRISING SUCH COMPOUNDS FOR THE PREVENTION OR TREATMENT OF DYSLIPIDAEMIAS

(30) Priorité: 08.10.2013 FR 1302334
(43) Date de publication de la demande: 14.09.2016
(73) Titulaire: Centre National de la Recherche Scientifique, 75016 Paris (FR); Universite De Poitiers, 86000 Poitiers (FR); University of Exeter, Exeter, Devon EX4 4QJ (GB)
(72) Inventeur: SPANOVA, Miroslava, 935 21 Tlmace-lipnik (SK); FERREIRA, Thierry, F-86240 Iteuil (FR); CLEMENT, Romain, F-Poitiers 86000 (FR); DHAYAL, Shalinee, Saltash Cornwall PL12 6XJ (GB); MORGAN, Noël, Gunnislake Cornwall PL18 9NF (GB)
(74) Mandataire: Cabinet Becker et Associés
(86) Numéro de dépôt international: PCT/FR2014/052546
(87) Numéro de publication internationale: WO 2015/052433

(56) Documents cités:
- EP-A2- 0 104 043
- WO-A1-2010/149170
- WO-A2-02/083059
- WO-A2-2012/054527
- STONE VIRGINIA M ET AL: "The cytoprotective effects of oleoylethanolamide in insulin-secreting cells do not require activation of GPR119.", BRITISH JOURNAL OF PHARMACOLOGY APR 2012, vol. 165, no. 8, avril 2012 (2012-04), pages 2758-2770, XP002721326, ISSN: 1476-5381
- MORGAN N G ET AL: "Unsaturated fatty acids as cytoprotective agents in the pancreatic <2>-cell", PROSTAGLANDINS LEUKOTRIENES AND ESSENTIAL FATTY ACIDS, CHURCHILL LIVINGSTONE, EDINBURGH, vol. 82, no. 4-6, 1 avril 2010 (2010-04-01), pages 231-236, XP027022275, ISSN: 0952-3278 [extrait le 2010-03-04]
- PINEAU LUDOVIC ET AL: "Lipid-induced ER stress: synergistic effects of sterols and saturated fatty acids.", TRAFFIC (COPENHAGEN, DENMARK) JUN 2009, vol. 10, no. 6, juin 2009 (2009-06), pages 673-690, XP002733951, ISSN: 1600-0854 cité dans la demande
- LAURIE-ANNE PAYET ET AL: "Saturated Fatty Acids Alter the Late Secretory Pathway by Modulating Membrane Properties", TRAFFIC, vol. 14, no. 12, 2 décembre 2013 (2013-12-02), pages 1228-1241, XP055159381, ISSN: 1398-9219, DOI: 10.1111/tra.12117
- Laurie-Anne Payet: "Effets des acides gras saturés sur la voie de sécrétion. Relation avec la mucoviscidose", Thèse Université de Poitiers , 29 novembre 2013 (2013-11-29), XP055159587, Extrait de l'Internet: URL:http://nuxeo.edel.univ-poitiers.fr/nux eo/site/esupversions/e25b5dd2-e52f-4642-a6 45-072e90abd59d [extrait le 2014-12-18]

## Description

La présente invention concerne le domaine de la médecine. Elle concerne plus particulièrement l'utilisation de composés pour prévenir et/ou traiter, chez un sujet, une dyslipidémie, qu'elle soit d'origine alimentaire ou, alternativement, associée à une hypoxie cellulaire, ladite dyslipidémie étant typiquement liée à la présence en excès dans les membranes biologiques, y compris dans les membranes biologiques de cellules non adipocytaires, d'acides gras, en particulier d'acides gras à longues chaînes saturées, et/ou de stérols. L'invention concerne également les compositions, en particulier les compositions pharmaceutiques et suppléments ou compléments alimentaires, comprenant de tels composés, ainsi que leurs utilisations pour prévenir et/ou traiter une dyslipidémie. Les composés et compositions selon l'invention peuvent en particulier être avantageusement utilisés pour prévenir et/ou traiter une pathologie choisie parmi le syndrome métabolique et/ou un symptôme ou une anomalie caractéristique du syndrome métabolique, de préférence pour prévenir et/ou traiter le diabète de type 2 (« T2DM ») ou la stéatose hépatique.

### ART ANTERIEUR

Insulino-résistance, insulino-déficience, hyperglycémie, hypercholestérolémie, en particulier hypercholestérolémie caractérisée par une faible concentration de cholestérol HDL, hypertriglycéridémie, hypertension, insuffisance cardiaque, et stéatose hépatique figurent au rang des symptômes ou anomalies caractéristiques du syndrome métabolique.
Le syndrome métabolique se définit notamment et typiquement (en l'absence de traitement) comme la manifestation d'au moins trois des cinq anomalies suivantes : obésité abdominale, hypertriglycéridémie (TG ≥ 1,7 mM environ), faible concentration de cholestérol HDL (HDLc < 1 mM environ pour les hommes et < 1,3 mM environ pour les femmes), hypertension (PA ≥ 130/85 mm Hg environ) et glycémie à jeun ≥ 5,5 mM environ (cf. « Syndrome métabolique et diabète chez l'homme. Composition lipidique et oxydation des lipoprotéines de basse densité (DL) plasmatiques en relation avec l'activation des plaquettes sanguines » - manuscrit de thèse de Romain Colas - soutenance du 10 décembre 2010).
L'implication des dyslipidémies dans le développement du syndrome métabolique est connue depuis plusieurs décennies.

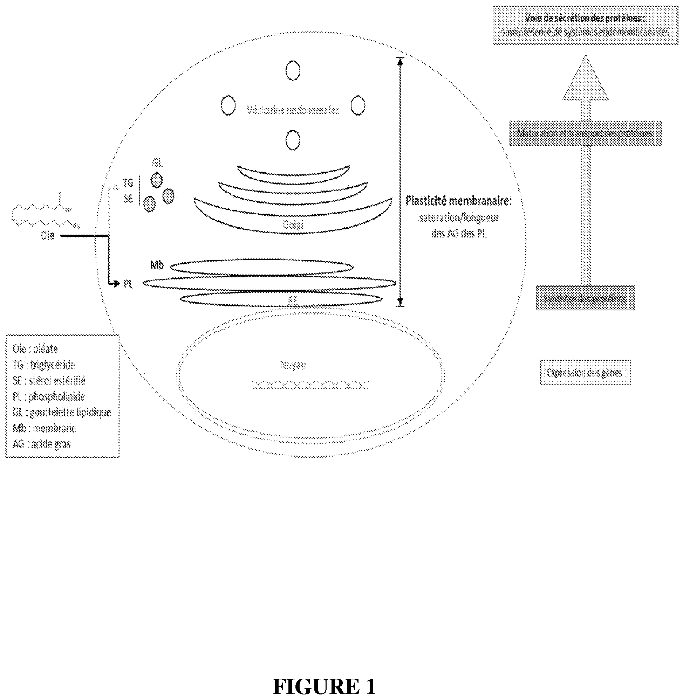

Une dyslipidémie se définit typiquement comme une concentration anormalement élevée ou diminuée de lipides, typiquement d'acides gras libres ou non estérifiés, stérols (par exemple cholestérol), triglycérides ou phospholipides, dans le sang. La plupart des dyslipidémies consistent en une augmentation du taux de ces éléments, une diminution étant beaucoup plus rare.
Les acides gras non estérifiés (AGNE) ou acides gras libres (AGL) représentent un élément énergétique important de l'organisme. Ils sont constitués d'un mélange complexe d'acides gras différant par leur nombre de double liaison et le nombre d'atomes de carbones composant leur chaîne hydrocarbonée. D'origine endogène, ils sont formés par biosynthèse dans le cytoplasme des cellules et sont utilisés pour la synthèse des triglycérides, sous forme d'acylcoA, dans le tissu adipeux et le foie, ou oxydés par les cellules. Ils entrent également dans la composition de lipides structuraux constituant les membranes biologiques, comme les phospholipides et les sphingolipides. Dans le plasma, on retrouve principalement quatre acides gras qui représentent 85 % des AGNE : acides oléique, palmitique, linoléique et stéarique. La majorité des AGNE est liée à l'albumine. Ils proviennent des triglycérides du tissu adipeux hydrolysés au cours du jeûne sous l'action de la lipoprotéine lipase tissulaire et sanguine en glycérol et acides gras. Leur concentration varie dans des proportions importantes en fonction de l'âge, de la prise de repas et de l'exercice physique. Généralement, en période post-prandiale, leur libération est supprimée.
Un stérol est un lipide possédant un noyau de stérane dont le carbone 3 est porteur d'un groupe hydroxyle. Les stérols sont considérés comme une sous-classe des stéroïdes. Le cholestérol, l'un des stérols les plus communs et répandus, est vital pour le fonctionnement cellulaire et est un précurseur de vitamines et d'hormones stéroïdiennes liposolubles.
Typiquement, à une concentration anormalement élevée de lipides dans le sang correspond une concentration anormalement élevée de lipides dans les membranes biologiques (« dyslipidémie cellulaire »). Par exemple, à une concentration anormalement élevée d'acides gras saturés libres (AGNE) dans le sang, correspond une concentration anormalement élevée d'acides gras saturés estérifiés (AGE) dans les phospholipides des membranes biologiques.
Ces lipides se trouvent toujours associés à des protéines spécifiques pour former des lipoprotéines. Les dyslipidémies résultent d'une dérégulation de l'homéostasie lipidique.
Il est établi qu'une alimentation excessivement riche en matières grasses d'origine animale conduit notamment à une accumulation d'acides gras saturés (AGS) dans les membranes biologiques (« lipo-intoxication ») et que ceci conduit à la perturbation globale de la plasticité membranaire au niveau cellulaire puis à l'inactivation métabolique des cellules et, à terme, à l'apoptose de la cellule (Figure 1).
Jusqu'à présent, seuls des acides gras insaturés (AGI), en particulier l'acide oléique (huile d'olive), étaient connus pour contrer les effets délétères d'une intoxication liée à l'accumulation d'AGS (Cunha *et al*., 2008 ; Diakogiannaki *et al*., 2008; Katsoulieris *et al*., 2009 ; Pineau *et al*., 2009 ; Stein *et al*., 1997 ; Wei *et al*., 2006 ; Deguil *et al*., 2011).
Cependant, leur utilisation en tant qu'alicament et/ou médicament rencontre deux limites majeures. D'une part, les AGI présentent essentiellement des propriétés préventives et ont donc un intérêt limité dans le cadre du traitement des lipo-intoxications installées, *i.e.* des lipo-intoxications responsables d'une perturbation de l'ensemble des mécanismes membranaires (détectable à toutes les étapes de la voie de sécrétion des protéines). En effet, les AGI agissent en entrant en compétition directe avec les AGS, lors de la prise alimentaire, dans la synthèse des phospholipides (PL) membranaires. D'autre part, la toxicité des AGI a été démontrée sur des cellules incapables de transformer (« tamponner ») puis de stocker l'excès d'acides gras libres, notamment d'AGI, en lipides neutres, typiquement en triglycérides (TG) et/ou stérols estérifiés. C'est le cas par exemple pour une souche de levure chez laquelle, du fait de l'absence des quatre enzymes acyltransferases Lrolp, Dgalp, Are1p et Are2p, une dérégulation de la synthèse des lipides neutres est observée. Lors d'une exposition de cette souche mutante à une source d'AGI exogènes, la dérégulation lipidique se traduit par une prolifération massive des membranes intracellulaires puis par la mort des cellules, par un processus indépendant de l'UPR (Unfolded Protein Response ; voir ci-après) (Kohlwein & Petschnigg, 2007; Petschnigg *et al*., 2011). De manière intéressante, des phénomènes identiques ont pu être observés dans des cellules de mammifères (Listenberger *et al*., 2003). Ceci explique pourquoi des acides gras insaturés deviennent toxiques pour la cellule dans des conditions de lipo-intoxication préalable de cette dernière, état dans lequel la capacité de stockage des acides gras insaturés sous la forme de lipides neutres, par la cellule, est dépassée (cellule lipo-intoxiquée qualifiée de « métaboliquement inactive »), ou, alternativement, dans des conditions normales, pour des cellules présentant une très faible capacité de synthèse de TG, telles que les cellules pancréatiques non-beta (Cnop M *et al*., 2001). Chez l'Homme, à l'exception des adipocytes (seuls capables de synthétiser des lipides neutres et de les stocker), l'ensemble des types cellulaires est ainsi susceptible d'être concerné par la lipo-intoxication. Il est notamment connu que les perturbations liées à l'accumulation d'AGS conduisent à l'apoptose des cellules β-pancréatiques responsables de la synthèse d'insuline (Butler *et al*., 2003) ou à celle des hépatocytes (Egnatchik *et al*., 2014).

Dans le cas du diabète de type 2 (« T2DM »), les répercussions de l'accumulation d'AGS se manifestent dans différents organes et se traduisent en particulier par une insulino-déficience au niveau du pancréas (liée à l'apoptose des cellules β-pancréatiques, décrite ci-dessus) mais également par une insulino-résistance au niveau du foie et au niveau des muscles.
On estime aujourd'hui à 382 millions le nombre d'individus diabétiques dans le monde. Si l'implication d'une dérégulation de l'homéostasie lipidique a été établie depuis plusieurs décennies, la plupart des traitements actuels se focalisent sur le taux d'insuline sécrétée ou le taux de sucre dans la circulation sanguine. Concrètement, plusieurs molécules sont employées pour le traitement du diabète de type 2. Celles-ci sont testées pour chaque patient puis séquentiellement remplacées par de nouvelles (en fonction de leur influence sur la masse corporelle et d'autres effets secondaires éventuels), si elles s'avèrent inefficaces. Selon les recommandations AFSSAPS de 2006, la metformine (un type de biguanide) est utilisée prioritairement afin de diminuer l'insulino-résistance sans provoquer d'hypoglycémie. Dans un second temps, des insulino-sécréteurs tels que des sulfamides hypoglycémiants ou des glinides peuvent être utilisés. Par ailleurs, depuis 2008, des inhibiteurs de DPP4 (gliptines) et autres analogues du GLP1 (incrétino-mimétiques) ont également fait leur apparition dans l'éventail des produits à disposition pour rectifier la glycémie sans pour autant s'intéresser au contexte dyslipidémique. Enfin, en dernier recours, des injections d'insuline sont prescrites.
Aucune des stratégies évoquées ne permet de restaurer, à la base, la fonctionnalité des cellules et organes lipo-intoxiqués et n'est donc en mesure d'intervenir au niveau d'étapes précoces dans la cascade des effets délétères rencontrés dans le syndrome métabolique ou encore dans le diabète de type 2, typiquement en amont de chacune des étapes ciblées par les traitements existants. La démarche thérapeutique contemporaine qui vise à stimuler les fonctions physiologiques d'organes « malades » pourrait même, de manière contre-productive, contribuer à leur affaiblissement et expliquer l'inefficacité des médicaments utilisés chez de nombreux patients et, de surcroît, l'apparition de phénomènes de résistance au cours du temps.

Les inventeurs décrivent à présent des molécules ou composés, et compositions comprenant de tels molécules ou composés, permettant de prévenir la survenue d'une dyslipidémie au sein des membranes biologiques, typiquement l'accumulation cellulaire d'acides gras, en particulier d'acides gras saturés, et/ou de stérols, ou de traiter une dyslipidémie installée en agissant sur le phénomène communément altéré dans l'ensemble des tissus lipo-intoxiqués : la plasticité membranaire.

### RESUME DE L'INVENTION

L'invention concerne une nouvelle classe de molécules destinée à la prévention ou au traitement des pathologies associées à une lipo-intoxication par les acides gras, typiquement par les acides gras saturés (AGS) et/ou par les stérols, en particulier par les acides gras à longue chaîne saturée et/ou trans. Par acides gras à longues chaînes on entend typiquement les acides gras dont la chaîne carbonée comprend au moins 14 atomes de carbone, typiquement entre 14 et 24 atomes de carbone, par exemple au moins 16 ou au moins 18 atomes de carbone, typiquement entre 14 et 22 ou entre 14 et 18 atomes de carbones.
La lipo-intoxication peut se manifester par une inversion du rapport acides gras saturés/acides gras insaturés (AGS/AGI) dans les phopholipides (PL) présents au sein des membranes biologiques, les AGS devenant majoritaires, voire remplaçant complètement les AGI. Les molécules de l'invention sont ainsi typiquement destinées à la prévention ou au traitement d'une dyslipidémie, du syndrome métabolique, d'un symptôme ou d'une anomalie caractéristique du syndrome métabolique, de préférence à la prévention ou au traitement du diabète de type 2 ou de la stéatose hépatique.

Un avantage considérable que présentent les molécules (ou composés) de l'invention, par rapport aux acides gras insaturés (AGI), en particulier l'acide oléique, utilisés dans l'art antérieur pour compenser un excès d'acides gras saturés (AGS), est que contrairement à ces derniers, elles n'engendrent aucune toxicité cellulaire, en particulier aucune toxicité sur les cellules incapables de synthétiser des lipides neutres, typiquement sur les cellules non-adipocytaires, par exemple sur les cellules pancréatiques. Les molécules de l'invention présentent un autre avantage majeur en ce que, contrairement aux AGI utilisés de manière préventive dans l'art antérieur, elles peuvent également être utilisées de manière thérapeutique en raison de leur aptitude à rétablir la fonctionnalité cellulaire, par exemple en agissant sur la plasticité membranaire. Elles peuvent ainsi avantageusement être utilisées pour traiter une dyslipidémie installée, *i.e.* une dyslipidémie responsable d'un dysfonctionnement cellulaire détectable, typiquement d'une altération de la capacité, voire d'une incapacité (inactivation métabolique), de la cellule à exercer sa fonction naturelle.

La description concerne ainsi un composé comprenant une tête polaire, comprenant au moins un résidu hydroxyle, sur laquelle est greffé un unique acide gras insaturé comprenant entre 16 et 24, par exemple entre 16 et 22 ou entre 16 et 20, atomes de carbone et ayant 1 à 6, par exemple 3, insaturation(s) en configuration *cis* pour une utilisation pour prévenir ou traiter une dyslipidémie chez un sujet. La dyslipidémie affecte typiquement les membranes biologiques, y compris les membranes biologiques de cellules non adipocytaires. Elle est généralement liée à la présence en excès dans lesdites membranes biologiques, d'acides gras saturés, plus particulièrement d'acides gras à longues chaînes saturées, et/ou de stérols. La quantité d'acides gras saturés et/ou de stérols est en particulier jugée excessive, par exemple, lorsqu'en altérant la plasticité membranaire elle provoque un dysfonctionnement cellulaire. Dans un mode de réalisation préféré de l'invention, ledit composé i) ne permet pas la production ou l'introduction de phospholipides di-insaturés dans la membrane des cellules traitées, typiquement ne restaure pas, chez la cellule traitée, une composition en acides gras des phospholipides membranaires comparable à celle des phospholipides membranaires d'une cellule correspondante non lipo-intoxiquée, ii) ne constitue pas une source d'acide oléique pour la cellule traitée, typiquement une source d'acide oléique capable de restaurer la plasticité membranaire de la cellule traitée, et de préférence iii) n'induit pas de mobilisation calcique intracellulaire et/ou n'est pas dégradé par les lipases.

La description concerne un composé dont la tête polaire est de formule (I) : 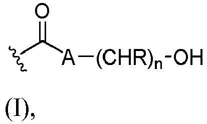 dans laquelle :
A est un atome d'azote ou d'oxygène, de préférence un atome d'oxygène,
n vaut 2 ou 3, de préférence n vaut 2, et
R est n'importe quel groupement chimique,
pour une utilisation pour prévenir ou traiter une dyslipidémie chez un sujet, typiquement une dyslipidémie telle que définie ci-dessus.

De tels composés pour leur utilisation dans la prévention et/ou le traitement d'une dyslipidémie sont décrits dans WO2012/054527, WO2010/149170 et EP0104043.

Les composés selon l'invention i) ne permettant pas la production de phospholipides di-insaturés, ou ne provoquant pas l'introduction de tels phospholipides dans la membrane des cellules traitées, typiquement ne restaurant pas, chez la cellule traitée, une composition en acides gras des phospholipides membranaires comparable à celle des phospholipides membranaires d'une cellule correspondante non lipo-intoxiquée, et ii) ne constituant pas une source d'acide oléique pour la cellule traitée, typiquement une source d'acide oléique capable de restaurer la plasticité membranaire de la cellule traitée, sont sélectionnés parmi le mannide monooléate, le 3-hydroxy-2,2-bis (hydroxyméthyl)propyl oléate et le N,N-diéthanololéamide (contrairement à l'acide 1 oléoyl lysophosphatidique ou LPA par exemple).

Les composés selon l'invention n'induisant pas de mobilisation calcique intracellulaire sont le mannide monooléate et le 3-hydroxy-2,2-bis (hydroxyméthyl)propyl oléate. Contrairement à l'OAG qui est une molécule de référence induisant une mobilisation calcique cellulaire (Marin & Cooper, 2006) ou, alternativement, au 1-oléoylglycérol et au 2-oléoylglycérol (Iwasaki et al., 2008), ces composés sont particulièrement avantageux en ce qu'ils présentent un risque de toxicité moindre par des processus délétères dépendant du taux de calcium intracellulaire, tels que la prolifération ou l'apoptose (Stutzmann GE et al., 2011).

Le composé selon l'invention préféré en ce qu'il résiste à l'action de dégradation des lipases est le N,N-diéthanololéamide.
L'invention concerne par ailleurs un composé tel que décrit dans le présent texte pour une utilisation pour prévenir ou traiter le syndrome métabolique, typiquement au moins un symptôme ou une anomalie caractéristique du syndrome métabolique, de préférence au moins deux ou trois symptômes, lesdits symptômes étant sélectionnés parmi insulino-résistance, insulino-déficience, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique, de préférence parmi insulino-résistance, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique. Un objet particulier concerne un composé selon l'invention pour une utilisation pour prévenir ou traiter le diabète de type 2.

Un composé selon l'invention particulièrement préféré est le mannide monooléate dont les inventeurs ont démontré, à l'aide de cellules beta-pancréatiques de mammifère lipointoxiquées par des acides gras saturés, qu'il est avantageusement capable d'augmenter la sécrétion d'insuline chez des sujets souffrant de lipointoxication et/ou de diabète de type 2 en favorisant chez eux la maturation de la pro-insuline en insuline.

L'invention concerne par ailleurs une composition, se présentant sous la forme d'une composition pharmaceutique, d'un alicament ou d'un supplément alimentaire, comprenant au moins un composé selon l'invention. Un objet particulier concerne typiquement une composition pharmaceutique comprenant outre ledit au moins un composé selon l'invention, au moins un autre composé (différent des composés selon l'invention) actif sur le plan thérapeutique (et reconnu comme tel par l'homme de l'art).

L'invention concerne également l'utilisation d'une telle composition pour prévenir ou traiter, chez un sujet, une dyslipidémie, typiquement une dyslipidémie dans les membranes biologiques, y compris dans les membranes biologiques de cellules non adipocytaires, en particulier une dyslipidémie liée à la présence en excès dans lesdites membranes biologiques, d'acides gras, plus particulièrement d'acides gras à longues chaînes saturées et/ou *trans*, et/ou de stérols. Elle concerne aussi l'utilisation d'une telle composition pour prévenir ou traiter, chez un sujet, le syndrome métabolique, typiquement au moins un symptôme ou une anomalie caractéristique du syndrome métabolique, de préférence plusieurs symptômes (par exemple 2, 3, 4 ou 5) ; de préférence pour prévenir ou traiter le diabète de type 2. Les utilisations décrites peuvent aussi être avantageusement mises en oeuvre en combinaison avec au moins un autre composé actif sur le plan thérapeutique (reconnu comme tel par l'homme de l'art et différent des composés selon l'invention) en particulier dans le traitement du syndrome métabolique, typiquement d'au moins un symptôme ou d'une anomalie caractéristique du syndrome métabolique, et/ou du diabète de type 2.

### DESCRIPTION DETAILLEE

Les inventeurs ont démontré que les AGS provenant d'une source exogène (alimentation) ou endogène (hypoxie ou altération, par mutation, des étapes de désaturation des acides gras) s'accumulaient au sein des phospholipides constituants les membranes cellulaires, perturbant ainsi de nombreux processus, en altérant la fonctionnalité des organelles intracellulaires intervenant dans la voie de sécrétion des protéines (cf. Figure 1).

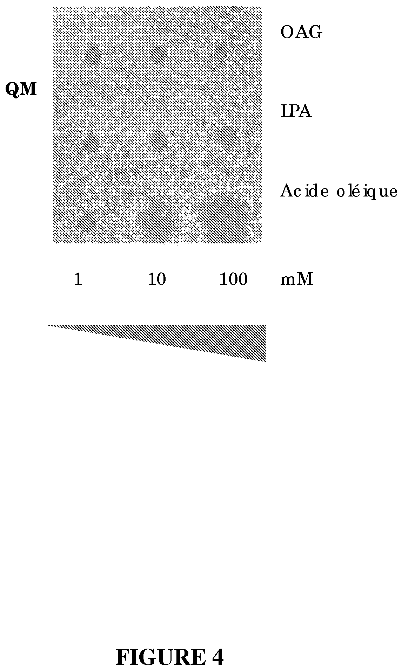

Pour apporter cette démonstration, les inventeurs ont développé un modèle unicellulaire simple (souche *hem1Δ* élaborée à partir de la levure de boulangerie *Saccharomyces cerevisiae*) reproduisant l'ensemble des impacts des AGS et du cholestérol observés dans les cellules de mammifères, en particulier l'ensemble des anomalies impliquées dans le développement du syndrome métabolique (Pineau *et al*., 2008 et Pineau *et al*., 2009).
En milieu YPG (i.e. milieu ne contenant ni Ergostérol (Erg) ni acide oléique (Ole)), la souche *hem1Δ* accumule des acides gras saturés (notamment l'acide palmitique, C16 :0) dans ses phospholipides, en particulier dans la phosphatidylcholine (PC). A noter que l'Ergostérol est le stérol majoritairement présent chez les levures et constitue donc chez les levures l'équivalent du cholestérol pour l'Homme.
La souche QM (Petschingg *et al*., 2009), dans laquelle les gènes codant pour les enzymes responsables de la synthèse des triglycérides et d'esters de stérols ont été délétés, est quant à elle incapable de transformer un apport exogène d'acides gras libres, type acide oléique C18 :1, en lipides neutres, de sorte que cet apport entraîne un stress délétère du fait de la perturbation de l'équilibre de la plasticité membranaire qu'il génère. L'utilisation de la souche QM a en particulier permis aux inventeurs de réaliser des tests de toxicité qui ont permis de démontrer clairement la toxicité de l'acide oléique dans de telles circonstances (cf. figure 4). Plus précisément, les inventeurs ont observé sur les souches *hem1Δ* les effets négatifs de l'accumulation de phospholipides portant des chaînes saturées (PL saturés) et de cholestérol dans les membranes des organelles intracellulaires, sur la formation des vésicules de sécrétion. Cette lipo-intoxication (endogène car le système cellulaire des souches *hem1Δ* ne synthétise plus que des AGS) perturbe l'environnement lipidique de la membrane du réticulum endoplasmique (RE), altère le processus de repliement des protéines (« misfolding ») et déclenche alors une réponse complexe dans ledit RE, réponse connue sous le nom de « Unfolded Protein Response » (UPR). Une saturation de ce système de sauvegarde entraine la mort cellulaire par apoptose. Parallèlement, les inventeurs ont pu observer des perturbations de la vésiculation de l'appareil de Golgi ainsi qu'une altération du trafic de protéines de référence (ex : Fur4p) entre l'appareil de Golgi et la membrane plasmique. Concrètement, les inventeurs ont observé une altération de l'ensemble de la voie de sécrétion, due à la lipo-intoxication. En d'autres termes, la souche *hem1Δ* de levure leur a permis de confirmer à la fois les impacts des AGS sur le stress du RE mais également sur le trafic des protéines vers la membrane plasmique.

Le réticulum endoplasmique (RE) est impliqué dans plusieurs processus cellulaires fondamentaux, incluant la synthèse lipidique, la régulation de l'homéostasie calcique et la synthèse des protéines destinées aux différents organites et à la surface cellulaire (par exemple les protéines membranaires telles que les canaux ioniques et les transporteurs). Le RE est également le site où les protéines membranaires ou sécrétées sont assemblées et repliées. En conséquences, l'UPR joue un rôle essentiel dans le maintien de l'intégrité et de la fonctionnalité du RE, en permettant à cet organite de gérer l'accumulation de protéines mal-repliées (Kincaid & Cooper, 2007; Zhang & Kaufman, 2006). A noter que la toxicité des AGS est associée, dans les cellules β-pancréatiques (responsables de la synthèse d'insuline chez les mammifères) à l'induction de la réponse UPR (Cunha *et al*., 2008; Diakogiannaki & Morgan, 2008; Laybutt *et al*., 2007). Alkhateeb *et al.* (2007) et Kato *et al.* (2008) ont en outre observé que l'accumulation d'AGS altère l'adressage du récepteur à l'insuline et du transporteur du glucose Glut4 à la surface des cellules musculaires.
Schneider *et al.* (1999) ont observé que les membranes du réticulum endoplasmique (RE) et de l'appareil de Golgi sont constituées très majoritairement de phospholipides (PL) insaturés, alors que le taux de PL saturés augmente graduellement dans les compartiments les plus distaux dans la voie de sécrétion pour atteindre son maximum à la membrane plasmique. Des taux importants de PL insaturés se traduisent par une fluidité membranaire élevée, un paramètre crucial pour le recrutement de certaines protéines essentielles à la formation des vésicules. Un exemple canonique est fourni par les protéines de la famille des Arf-GAP1, l'une d'entre-elle étant Gcs1p chez la levure. Il a été montré que Gcs1p est un médiateur du transport vésiculaire à la fois entre l'appareil de Golgi et le RE, et entre le RE et la membrane plasmique (Robinson *et al*., 2006). De façon intéressante, la délétion du gène GCS1 provoque une fragmentation de l'appareil de Golgi et une perturbation du trafic vésiculaire post-Golgien (Poon *et al*., 2001), autant de phénomènes que les inventeurs ont eux-mêmes pu constater dans le modèle de levure *hem1Δ*, *i.e.* en condition d'accumulation d'AGS (cf. Payet *et al*., 2013.).
Les protéines de la famille Arf-GAP1 répondent à la courbure membranaire en s'adsorbant à la surface membranaire *via* un motif spécifique appelé ArfGAP1 Lipid Packing Sensor (ALPS; (Bigay *et al*., 2005)). Concrètement, le motif ALPS ne reconnaît pas la courbure membranaire *per se,* c'est-à-dire une géométrie courbe, mais reconnaît un faible empilement des têtes polaires des phospholipides (« Loose Lipid Packing ») qui est une conséquence de la courbure membranaire (Bigay *et al*., 2005). Les inventeurs sont parvenus à démontrer que les taux élevés de PL saturés en conditions de lipo-intoxication sont associés à une augmentation du Lipid Packing membranaire (Deguil *et al*., 2011), et que cette augmentation altère le recrutement par l'appareil de Golgi du Gcs1p en provenance du cytoplasme (Payet *et al*., 2013). Plus généralement, ils ont démontré que l'accumulation d'acides gras, en particulier d'AGS, dans les membranes biologiques provoquait la dérégulation fonctionnelle des organelles ou organites intra-cellulaires dont l'appareil de Golgi et le Réticulum Endoplasmique (RE), et en particulier une diminution du taux de vésiculation responsable d'une diminution de la translocation de certains transporteurs et récepteurs membranaires à la surface cellulaire.

Les lipo-intoxications cellulaires provoquées par les inventeurs résultent, *in vitro*, d'une exposition à une source exogène d'acides gras exclusivement sous forme saturée (lipo-intoxication « exogène ») ou, alternativement, d'une incapacité intrinsèque de la cellule à produire des formes insaturées des acides gras (lipo-intoxication « endogène »).

A l'aide de leur modèle de levure *hem1Δ*, les inventeurs ont montré que l'acide oléique (Ole), en étant métabolisé dans les phospholipides (PL) (cf. figure 1 - perte de PL à AGS en faveur de PL à AGI), permet de restaurer la plasticité de membranes préalablement lipo-intoxiquées par des AGS. Ils ont également démontré à l'aide de la souche de levure QM que l'effet bénéfique observé se limitait aux cellules ayant la capacité de tamponner un excès d'AGI exogènes sous forme de lipides neutres. Dans les cellules n'ayant pas cette capacité, le surplus d'acide oléique exogène entraîne, *in fine*, une prolifération anormale des membranes intracellulaires laquelle, en stressant les cellules, va déclencher leur apoptose.

Les inventeurs ont utilisé leur modèle de levure *hem1Δ* et la souche QM pour cribler des molécules d'intérêt susceptibles d'empêcher ou de limiter ce phénomène, idéalement de contrer l'effet toxique des acides gras présents en excès et/ou mal métabolisés (*i.e.* estérifiés) toxiques et de corriger l'ensemble des phénomènes perturbés. Ils ont ainsi découvert des molécules capables, en particulier, de restaurer une fonctionnalité cellulaire (en restaurant par exemple la fluidité membranaire) comparable à celle rencontrée dans des conditions non pathologiques.

L'efficacité des molécules présélectionnées par les inventeurs, *i.e.* leur capacité à restaurer une fonctionnalité cellulaire comparable à celle rencontrée dans des conditions non pathologiques, même dans le cas de dyslipidémies installées, a ensuite été testée et démontrée par ces mêmes inventeurs sur des cellules β-pancréatiques de mammifère, en particulier dans des cellules β-pancréatiques de rat (lignée BRIN-BD11). Par ailleurs, les inventeurs ont pu mettre en évidence que les composés d'intérêt présentent une influence très limitée sur des phénomènes cellulaires tels que la mobilisation calcique responsable de l'induction de phénomènes de prolifération cellulaire et d'apoptose. Ils présentent ainsi une toxicité moindre par rapport aux composés, tel que l'OAG, induisant ou favorisant, au contraire, une telle mobilisation calcique cellulaire. De même, certains composés se sont montrés particulièrement efficaces pour restaurer la conversion de la pro-insuline en insuline dans des cellules β-pancréatiques de mammifère, en particulier dans des cellules β-pancréatiques de souris (lignée MIN6).

L'invention porte ainsi sur un composé comprenant une tête polaire, comprenant au moins un résidu hydroxyle, sur laquelle est greffé un unique acide gras insaturé comprenant entre 16 et 24, par exemple entre 16 et 20, typiquement 18, atomes de carbone et ayant 1 à 6, par exemple 3, insaturation(s) en configuration *cis* (identifié dans le présent texte en tant que « composé d'intérêt ») pour une utilisation pour prévenir ou traiter une dyslipidémie chez un sujet.
Le sujet concerné est un animal, typiquement un mammifère, par exemple un mammifère choisi parmi une souris, un rat, un porc et un être humain. Le sujet concerné est de préférence un être humain.

Dans le contexte de la présente description, la dyslipidémie dont la prévention ou le traitement est recherchée affecte typiquement les membranes biologiques, en particulier les membranes biologiques de cellules non adipocytaires. Elle est généralement liée à la présence en excès dans lesdites membranes biologiques, d'acides gras, plus particulièrement d'acides gras à longues chaînes saturées et/ou *trans*, et/ou de stérols. La dyslipidémie est typiquement responsable de l'intoxication (lipo-intoxication) des cellules non adipocytaires à l'origine du dysfonctionnement ou de l'apoptose desdites cellules par diminution, voire suppression, de la fluidité de leur membrane plasmique et/ou de la membrane de leurs organelles.
Dans un mode de réalisation particulier de l'invention, la dyslipidémie est associée à la présence chez le sujet d'un syndrome métabolique, typiquement d'au moins un symptôme du syndrome métabolique, de préférence au moins deux ou trois symptômes, lesdits symptômes étant sélectionnés parmi insulino-résistance, insulino-déficience, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique, de préférence parmi insulino-résistance, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique.

Ainsi qu'il ressort de la présente description, l'expression « présence en excès » d'acides gras, en particulier d'AGS, et/ou de stérols, est synonyme de « lipo-intoxication », par exemple de lipo-intoxication d'origine exogène ou, alternativement, de lipo-intoxication d'origine endogène (par exemple hypoxique), et désigne la présence, dans une cellule non adipocytaire, en particulier d'acides gras saturés et/ou *trans*, et/ou de stérols en une quantité suffisante pour perturber la voie de sécrétion décrite plus haut et ainsi altérer le fonctionnement cellulaire (typiquement la voie de sécrétion des protéines et en conséquence la fonction desdites protéines), voire, à un niveau supérieur, altérer en conséquence le fonctionnement de l'organe correspondant.
Une lipo-intoxication rénale se manifeste par exemple lorsque des acides gras saturés, en particulier à chaîne longue, sont stockés dans les cellules du rein et les cellules du tube contourné proximal. Un tel stockage conduit à une inflammation tubulo-interstitielle et à une fibrose, voire à une insuffisance rénale, et à la mort du sujet concerné dans les cas les plus sévères. Encore à titre d'exemple, une lipo-intoxication du pancréas se diagnostique typiquement par le stockage au sein des phospholipides membranaires d'acides gras saturés, en particulier à chaîne longue, dans les cellules β-pancréatiques.
A l'échelle cellulaire, une lipo-intoxication se diagnostique typiquement par la mise en évidence d'une modification du contenu en acides gras des phospholipides (PL) des membranes biologiques (au niveau des espèces phospholipidiques de phosphatidylcholine (PC) notamment) et, en particulier, par la déplétion des formes de PL à AGI au profit de PL à AGS. A l'image du mode opératoire décrit dans la partie expérimentale de la présente description, une telle signature lipidomique peut être mise en évidence suite à l'extraction des lipides cellulaires totaux, à la purification de leurs phospholipides et à l'analyse de ces derniers par spectrométrie de masse (Deguil *et al*., 2011).
Par ailleurs, cette lipo-intoxication cellulaire peut se manifester par l'induction de la réponse UPR (« Unfolded Protein Response »). Ainsi que le démontre la partie expérimentale, il est possible, *in vitro*, de détecter et de mesurer cette réponse UPR par l'analyse de l'expression d'un gène rapporteur (tel que le gène *lacZ* codant pour la β-galactosidase dont l'activité enzymatique peut être quantifiée) contenant dans sa séquence promotrice un ou des, par exemple 4, élément(s) de réponse à l'UPR (« UPRE ») spécifique(s) d'un gène caractéristiquement induit lors du déclenchement de ladite réponse, par exemple d'un gène choisi parmi CHOP, BiP, GRP78 et ATF4 (Laybutt *et al*., 2007). Alternativement, le déclenchement de l'UPR en réponse à une lipo-intoxication peut être détecté et mesuré en quantifiant la proportion de formes actives de certaines protéines clés dans cette cascade d'événements cellulaires. C'est le cas de la protéine eIF2α dont l'abondance de la forme active phosphorylée est proportionnelle à l'état d'activation de l'UPR. Comme expliqué dans la partie expérimentale, la quantité de la forme active phosphorylée peut être évaluée par densitométrie des images obtenues après western blot (Dhayal & Morgan, 2011).
Dans le contexte de la présente invention, la réponse UPR peut être avantageusement détectée ou mesurée par détection ou mesure de l'expression d'un gène ou de l'activité d'une protéine impliqué(e) dans la réponse « UPR », comme expliqué ci-dessus.

Un composé décrit dans le présent texte est un composé tel que défini précédemment dont la tête polaire est de formule (I) : 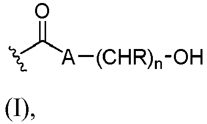 dans laquelle :
A est typiquement un atome d'oxygène ou un groupe NR₁, avec R₁ = H ou un alkyle en C₁-C₆ éventuellement substitué par un OH, et A est de préférence un atome d'oxygène ou NH ou
NCH₃ ou NCH₂CH₂OH et de manière encore plus préférée A est un atome d'oxygène,
n = 2 ou 3, de préférence n = 2, et
R est n'importe quel groupement chimique et peut être différent d'un groupement (CHR) à l'autre.

Dans la formule (I), la liaison interrompue par des zigzags représente la liaison entre la tête polaire et la chaîne carbonée de l'acide gras insaturé, le groupement C=O de la formule (I) étant le C=O de l'acide gras insaturé.
De préférence, R est un groupement comprenant uniquement des atomes de carbone, d'hydrogène et d'oxygène.
De préférence, R est un groupement saturé comprenant uniquement des atomes de carbone, d'hydrogène et d'oxygène.
De préférence, le radical (CHR)ₙ-OH est un dérivé du glycérol, de l'érythritol ou d'un monosaccharide tel que le mannose.
Dans la présente description, chaque résidu hydroxyle peut être indépendamment phosphaté.

Des exemples de composés décrits dans le présent texte comme utilisables pour prévenir ou traiter une dyslipidémie sont identifiés ci-dessous : 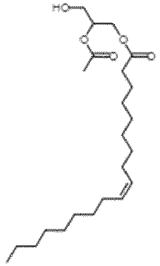 1-oléoyl-2-acétyl-sn-glycérl (**OAG**), 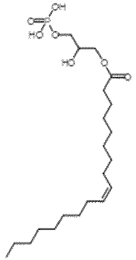 1-oléoyl-sn-glycérol-3-phosphate (acide 1-oléoyl lysophosphatidique ou **LPA**), 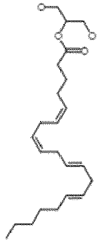 2-arachidonoylglycérol (**2-AG**), 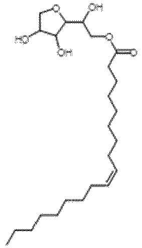 mannide monooléate, 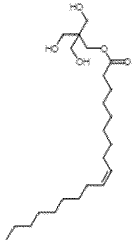 3-hydroxy-2,2-bis(hydroxyméthyl)propyl oléate, 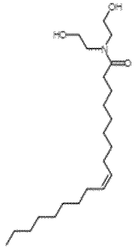 N,N-diéthanololéamide, 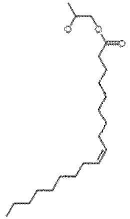 propylène glycol monooléate, 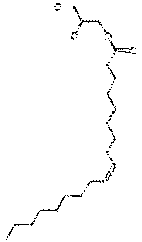 1-oléoyl glycérol, 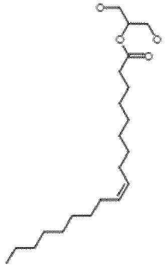 2-oléoyl glycérol, 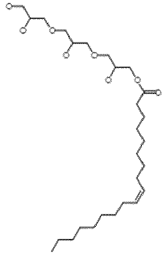 monoester d'acide oléique avec triglycérol, 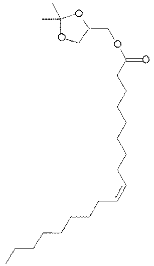 (Z)-9-Octadecenoic Acid -(2,2-Dimethyl-1,3-dioxolan-4-yl)methyl Ester 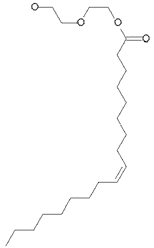 Diéthylène glycol monooléate

Des composés décrits comme utilisables pour prévenir ou traiter une dyslipidémie sont choisis par exemple parmi le 1-oléoyl-2-acétyl-sn-glycérol (OAG), le 1-oléoyl-sn-glycérol-3-phosphate (acide 1-oléoyl lysophosphatidique ou LPA), le 2-arachidonoylglycérol (2-AG), le mannide monooléate, le 3-hydroxy-2,2-bis(hydroxyméthyl)propyl oléate, le N,N-diéthanololéamide, le propylène glycol monooléate, le monoester d'acide oléique avec triglycérol et le (Z)-9-Octadecenoic Acid -(2,2-Dimethyl-1,3-dioxolan-4-yl)methyl Ester.

Les composés utilisés selon l'invention pour prévenir ou traiter une dyslipidémie, en particulier une lipo-intoxication associée à une maladie métabolique telle que le diabète de type II et/ou une lipo-intoxication hypoxique, sont choisis parmi le mannide monooléate, le 3-hydroxy-2,2-bis(hydroxyméthyl)propyl oléate et le N,N-diéthanololéamide.
Un composé d'intérêt particulièrement préféré pour prévenir ou traiter une dyslipidémie, en particulier dans le contexte de la prévention et/ou du traitement d'une maladie métabolique, et/ou d'un symptôme ou d'une anomalie caractéristique du syndrome métabolique, de préférence du diabète de type 2, est le mannide monooléate.

Les composés d'intérêt sont utilisés dans le contexte de l'invention pour prévenir ou traiter les dyslipidémies, typiquement en restaurant la fluidité des membranes biologiques. Une caractéristique avantageuse de ces composés est que, à la différence des acides gras insaturés utilisés dans l'art antérieur, ils sont non toxiques pour les cellules incapables de synthétiser des lipides neutres, typiquement des triglycérides et/ou stérols estérifiés. Ces composés sont en particulier non toxiques pour les cellules pancréatiques (cellules β-pancréatiques et cellules α-pancréatiques). Ils sont également de préférence non toxiques pour les cellules rénales, hépatiques, cardiaques et musculaires. Ils sont en outre de préférence avantageusement capables de restaurer la fonctionnalité d'une cellule lipo-intoxiquée ainsi que, le cas échéant, celle de l'organe impliqué.

Un composé d'intérêt typique de l'invention présente avantageusement les propriétés suivantes :
(i) il restaure la croissance d'un mutant *hem1Δ* de levure *Saccharomyces cerevisiae* lipointoxiqué,
(ii) il réduit ou supprime la réponse UPR (« Unfolded Protein Response »),
(iii) il n'est pas toxique pour un mutant QM de levure *Saccharomyces cerevisiae*,
   et/ou
(iv) il réduit ou supprime la mort cellulaire par apoptose d'une cellule de mammifère lipo-intoxiquée.
Des composés particuliers utilisés dans le contexte de l'invention sont capables de restaurer la croissance d'un mutant *hem1Δ* de levure *Saccharomyces cerevisiae* lipointoxiqué et/ou de réduire ou supprimer la réponse UPR (« Unfolded Protein Response »), typiquement la réponse UPR induite par une lipo-intoxication (que cette dernière soit de nature endogène ou exogène).

Des composés particuliers utilisés dans le contexte de l'invention sont non toxiques pour les levures de souche QM.

Des composés particuliers utilisés dans le contexte de l'invention sont en mesure de réduire ou supprimer la mort cellulaire par apoptose de cellules de mammifère lipo-intoxiquées.

Parmi les composés décrits dans le présent texte, certains agissent directement sur le contenu lipidique, *i.e.* sur la composition en acides gras, des phospholipides présents au sein des membranes cellulaires. Des exemples de tels composés sont le 1-oléoyl-sn-glycérol-3-phosphate (acide 1-oléoyl lysophosphatidique ou LPA) et le propylène glycol monooléate. Les composés décrits restaurent la fluidité et donc la fonctionnalité membranaire sans rétablir une composition normale en phospholipides di-insaturés au sein des membranes cellulaires. Un exemple préféré d'un tel composé est le 1-oléoyl-2-acétyl-sn-glycérol (OAG). Des exemples encore plus préférés sont le mannide monooléate, le 3-hydroxy-2,2-bis(hydroxyméthyl)propyl oléate et le N,N-diéthanololéamide.

Dans un mode de réalisation préféré de l'invention, les composés sont utilisés pour prévenir et/ou traiter une pathologie choisie parmi le syndrome métabolique et/ou un symptôme ou une anomalie caractéristique du syndrome métabolique, de préférence pour prévenir ou traiter le diabète de type 2.

Dans un mode de réalisation particulier de l'invention, les composés sont utilisés pour prévenir et/ou traiter le syndrome métabolique, typiquement au moins un symptôme du syndrome métabolique, de préférence au moins deux ou trois symptômes, lesdits symptômes étant sélectionnés parmi insulino-résistance, insulino-déficience, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique, de préférence parmi insulino-résistance, hyperglycémie (typiquement glycémie à jeun ≥ 5,5 mM environ), hypercholestérolémie, en particulier hypercholestérolémie caractérisée par une faible concentration de cholestérol HDL (typiquement < 1 mM environ pour les hommes et < 1,3 mM environ pour les femmes), hypertriglycéridémie (typiquement TG ≥ 1,7 mM environ), hypertension (typiquement pression artérielle (PA) ≥ 130/820 mm Hg environ), insuffisance cardiaque, et stéatose hépatique.

Comme expliqué précédemment, pour le diabète de type 2, les approches thérapeutiques actuelles ciblent des paramètres intervenant en aval des dyslipidémies initiales. Bien que validés dans des contextes physiologiques en laboratoire, ces traitements souffrent d'un manque d'efficacité lié à la perturbation globale des mécanismes membranaires constatée dans les cas de lipo-intoxication installées se manifestant en particulier par une fluidité membranaire altérée ou inefficace (en ce que la cellule concernée n'est plus fonctionnelle). Il n'existe en particulier à ce jour aucune molécule ou composé permettant de traiter les dyslipidémies affectant les cellules incapables de synthétiser des lipides neutres, en particulier les cellules non adipocytaires.

Dans un mode de réalisation préféré de l'invention, au moins un composé tel que décrit dans le présent texte est utilisé pour prévenir et/ou traiter le diabète de type 2. Le mannide monooléate est un exemple de composé utilisé de manière préférée pour prévenir et/ou traiter le diabète de type 2.
Ce au moins un composé peut être utilisé, dans un mode de réalisation particulier de l'invention, en combinaison avec un composé distinct connu de l'homme du métier et utilisé classiquement dans la prévention ou le traitement du diabète de type 2, ledit composé distinct étant de préférence choisi parmi biguanide, glitazone, sulfamide hypoglycémiant, glinide, inhibiteur de DPP4, incrétino-mimétique et inhibiteur d'α-Glucosidase.

Un autre objet de l'invention concerne par ailleurs une composition se présentant sous la forme d'une composition pharmaceutique, d'un alicament, d'un supplément ou d'un complément alimentaire, comprenant au moins un composé selon l'invention.
Un objet particulier concerne typiquement une composition pharmaceutique comprenant outre ledit au moins un composé selon l'invention, au moins un autre composé (différent des composés utilisés dans le contexte de l'invention pour prévenir ou traiter une dyslipidémie sans induire de toxicité sur les cellules non-adipocytaires) actif sur le plan thérapeutique (et reconnu comme tel par l'homme de l'art), en particulier un composé actif dans la prévention ou le traitement d'un symptôme ou d'une anomalie caractéristique du syndrome métabolique, et/ou du diabète de type 2 (tels que décrits dans le présent texte par exemple).

L'invention concerne également une composition telle que décrite dans le présent texte pour une utilisation pour prévenir ou traiter une dyslipidémie, typiquement une pathologie choisie parmi le syndrome métabolique et/ou un symptôme ou une anomalie caractéristique du syndrome métabolique, de préférence pour prévenir ou traiter le diabète de type 2.

Le terme « traitement » désigne le traitement curatif, symptomatique ou préventif. Les composés de la présente invention peuvent ainsi être utilisés chez des sujets (comme les mammifères, en particulier humains) atteints d'une maladie déclarée. Les composés de la présente invention peuvent aussi être utilisés pour retarder ou ralentir la progression ou prévenir une progression plus en avant de la maladie, améliorant ainsi la condition des sujets. Les composés de la présente invention peuvent enfin être administrés en « prévention » aux sujets non malades, mais qui pourraient développer normalement la maladie ou qui ont un risque important de développer la maladie.

Le ou les composés ou compositions selon l'invention peuvent être administrés de différentes manières et sous différentes formes.
Ainsi, dans un mode de réalisation typique, le ou les composés sont administrés au sujet, ensemble ou séparément, et le ou les composés ou compositions selon l'invention sont administrés de manière continue ou séquentielle, une ou plusieurs fois par jour (administration quotidienne), une ou plusieurs fois par semaine (administration hebdomadaire), ou une ou plusieurs fois par mois (administration mensuelle), pendant toute la durée du traitement, *i.e.* jusqu'à l'amélioration des symptômes de la pathologie traitée, de préférence la disparition de tout ou partie desdits symptômes.
Si nécessaire, la dose journalière peut par exemple être administrée en deux, trois, quatre, cinq, six ou plus, prises par jour ou par sous-doses multiples administrées par intervalles appropriés pendant la journée.
Ces composés ou compositions peuvent être par exemple administrés de manière systémique, par voie orale, parentérale, par inhalation ou par injection, comme par exemple par voie intraveineuse, intra-péritonéale, intra-musculaire, sous-cutanée, trans-dermique, intra-artérielle, etc. S'agissant d'un traitement à long terme, la voie d'administration préférée sera sublinguale, orale, intra-péritonéale, ou transcutanée.
Les compositions peuvent être formulées sous forme de suspensions injectables, huiles, suppositoires, gélules, capsules, aérosols, etc., éventuellement au moyen de formes galéniques ou de dispositifs assurant une libération prolongée et/ou retardée. Pour les injections, les composés sont généralement conditionnés sous forme de suspensions liquides, qui peuvent être injectées au moyen de seringues ou de perfusions, par exemple.
Il est entendu que le débit et/ou la dose injectée peuvent être adaptés par l'homme du métier en fonction du patient, de la pathologie, du mode d'administration, etc. En général, la dose journalière du composé sera la dose minimum pour obtenir l'effet thérapeutique.
La quantité de composé présente dans la composition thérapeutique peut être modulée de façon à obtenir un taux circulant de principe actif nécessaire à l'obtention de l'effet thérapeutique désiré pour un patient particulier, une composition, un mode d'administration, et ce de préférence sans toxicité pour le patient. La quantité choisie dépendra de multiples facteurs, en particulier de la voie d'administration, de la durée d'administration, du moment de l'administration, de la vitesse d'élimination du composé, du ou des différents produits utilisés en combinaison avec le composé, de l'âge, du poids et de la condition physique du patient, ainsi que de son histoire médicale, et de toutes autres informations connues en médecine.
Typiquement, les composés sont administrés à des doses pouvant varier entre 1 µg et 2 g par administration, préférentiellement de 0,1 mg à 1 g par administration. D'autre part, les compositions selon l'invention peuvent comprendre, en outre, d'autres agents ou principes actifs comme expliqué précédemment. Les compositions selon l'invention peuvent aussi comprendre un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc.

Sont aussi décrites dans le présent texte les méthodes de prévention ou de traitement d'une dyslipidémie chez un sujet comprenant l'administration à un sujet souffrant d'une dyslipidémie ou susceptible de développer une dyslipidémie d'un composé ou d'une composition d'intérêt tels que décrits dans le présent texte pour prévenir ou traiter ladite dyslipidémie.
La description concerne par ailleurs les méthodes de prévention ou de traitement chez un sujet atteint d'une pathologie choisie parmi syndrome métabolique, symptôme ou anomalie caractéristique du syndrome métabolique, et diabète de type 2. Ces méthodes comprennent toutes une étape d'administration à un sujet souffrant d'une telle pathologie ou susceptible de développer une telle pathologie d'un composé ou d'une composition d'intérêt tels que décrits dans le présent texte pour prévenir ou traiter ladite pathologie.

Les figures et exemples suivants illustrent l'invention sans en limiter la portée.

### LEGENDES DES FIGURES

**Figure 1** **: Voie de sécrétion et plasticité membranaire**
   Suite à leur synthèse, les protéines membranaires ou sécrétées (« outils » moléculaires des cellules) doivent subir des étapes de maturation à l'intérieur des cellules. Chacune des étapes de ce processus nommé « voie de sécrétion » a lieu dans un compartiment subcellulaire spécifique (réticulum endoplasmique (RE) et appareil de Golgi notamment). L'obtention de protéines matures nécessite donc un transport intracellulaire fonctionnel entre les différents systèmes endomembranaires. Ce flux est influencé, entre autres, par la plasticité des membranes des compartiments intracellulaires qui est, elle-même, directement corrélée à la nature des phospholipides (PL) qui composent les membranes. En particulier, il est admis que la présence d'acides gras saturés (AGS) dans les PL diminue la fluidité membranaire alors que des PL arborant des acides gras insaturés (AGI) forment des membranes plus fluides.
   L'effet bénéfique de l'acide oléique (Ole) est observé sur les cellules ayant la capacité de tamponner un excès d'AGI exogènes sous forme de lipides neutres (triglycérides (TG) ou stérols estérifiés (SE) stockés sous forme de gouttelettes lipidiques (GL)). Dans les cellules n'ayant pas cette capacité, le surplus d'oléate exogène entraîne, in fine, une prolifération des membranes intracellulaires laquelle en provoquant un stress cellulaire va déclencher l'apoptose.

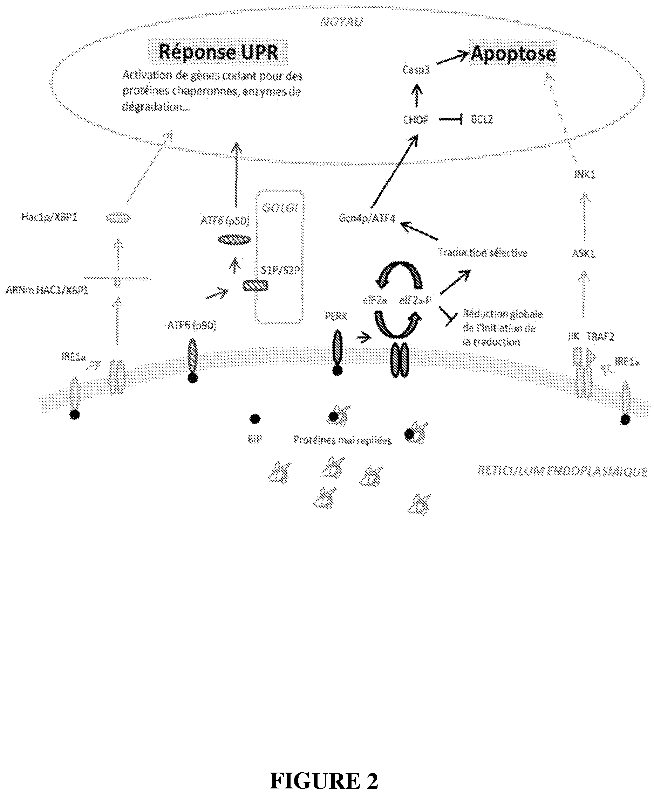

**Figure 2** **: Les voies de l'UPR chez les eucaryotes supérieurs** (Pineau & Ferreira, 2010).

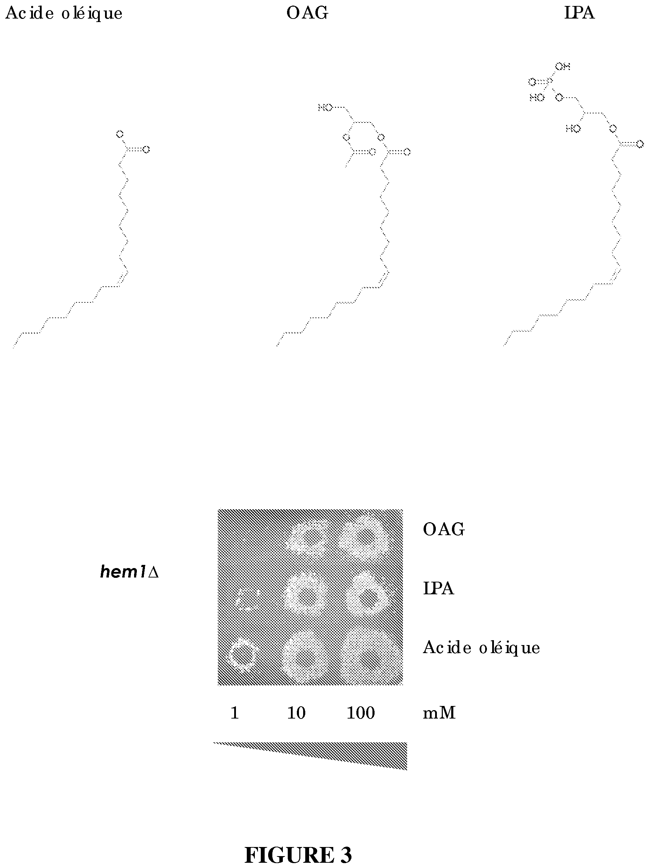

**Figure 3** **: L'acide oléique, l'OAG et le LPA restaurent la croissance de levures lipo-intoxiquées.**
   A) Structures moléculaires de l'acide oléique, de l'OAG et du LPA. B) Restauration de la croissance (après 3 jours) de levures *hem1Δ* cultivées dans des conditions d'accumulation d'AGS, en présence de concentrations croissantes d'acide oléique, d'OAG et de LPA.
**Figure 4** **: L'OAG et le LPA ne sont pas toxiques pour des cellules ne synthétisant pas de triglycérides.**
   Des gouttes de 5 µl d'OAG, de LPA ou d'acide oléique ont été déposées, à partir de solutions stock aux concentrations indiquées, à la surface d'un milieu gélosé sur lequel avait été préalablement étalée la souche QM. Après trois jours, des halos d'inhibition de croissance (absence de colonies) peuvent être observés dans le cas de l'acide oléique. Ces halos ne sont en revanche pas observés en présence de LPA ou d'OAG.

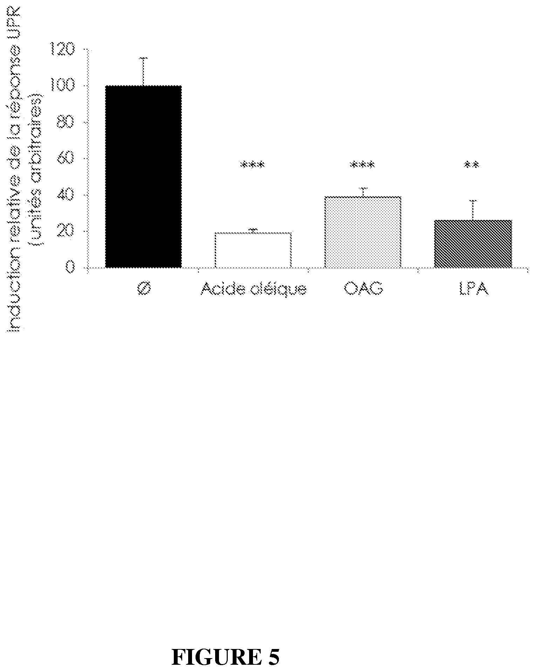

**Figure 5** **: L'OAG et le LPA réduisent la réponse UPR dans des levures lipointoxiquées.** Une construction plasmidique portant un gène de fusion, correspondant à la séquence codante du gène *LacZ* placée sous dépendance d'un promoteur artificiel contenant 4 éléments UPR (UPRE), a été introduite dans une souche *hem1Δ* de levure, comme décrit par Pineau *et al.* (2009). Lors d'une induction de la réponse UPR, le facteur de transcription Hac1p/XBP1p est activé et se fixe sur les éléments UPR du gène de fusion, entraînant la transcription du gène *LacZ. LacZ* codant pour la β-galactosidase, le niveau d'induction de l'UPR est donc mesuré par détection de l'activité enzymatique correspondante. La souche *hem1Δ* de levure a été cultivée dans un milieu liquide induisant l'accumulation d'AGS sans autre addition (ø), ou dans le même milieu supplémenté de 200 µM d'acide oléique, d'OAG ou de LPA, comme indiqué.

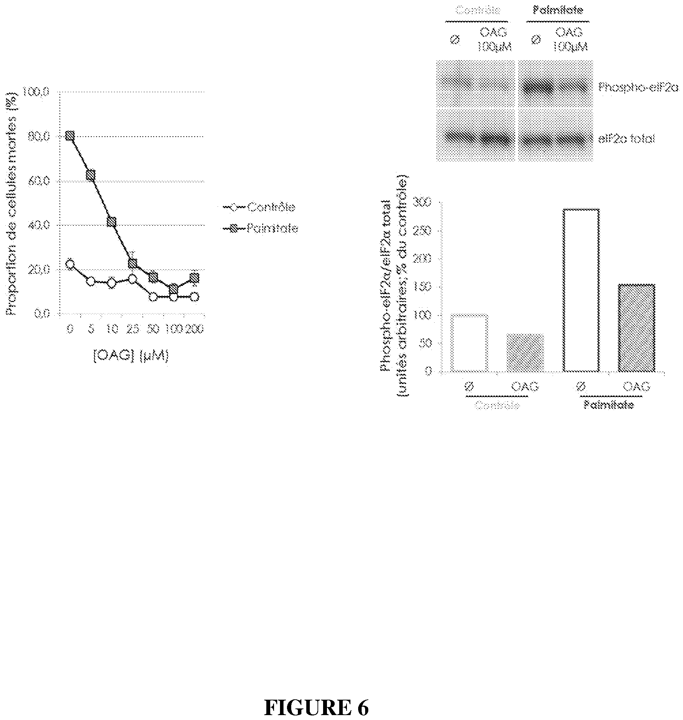

**Figure 6** **: L'OAG prévient l'apoptose des cellules β-pancréatiques en présence d'acides gras saturés, en réduisant le taux d'induction de l'UPR.**
   Des cellules β-pancréatiques BRIN-BD11 ont été cultivées dans des conditions contrôle ou en présence d'une source exogène d'acide gras saturé (acide palmitique, 200 µM), comme décrit par Dhayal & Morgan (2011) afin de générer des conditions de lipo-intoxication, avec ou sans addition d'OAG. A) La proportion de cellules mortes a été estimée en absence (contrôle) ou en présence d'acide palmitique, pour des concentrations croissantes d'OAG. B) Les taux de phosphorylation d'eIF2α ont également été analysés dans les différentes conditions par western blot, en présence ou en absence (Ø) d'OAG et normalisés aux quantités d'eIF2α totales. Le taux de phosphorylation étant corrélé à l'intensité de la réponse UPR, cette expérience démontre que l'OAG réduit l'UPR induite par l'accumulation d'acide palmitique.

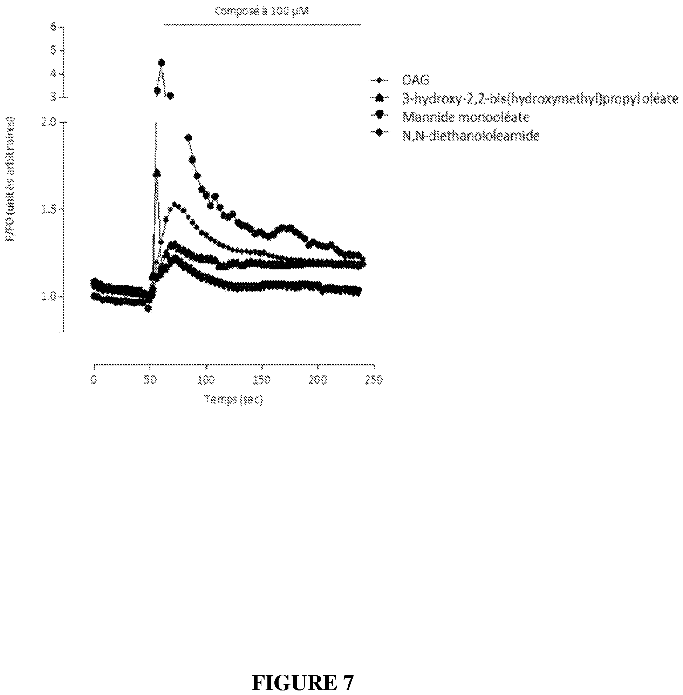

**Figure 7** **: Le 3-hydroxy-2,2-bis(hydroxyméthyl)propyl oléate et le mannide monooléate n'induisent pas de mobilisation calcique.**
   Les cellules épithéliales humaines, CFBE, ont été chargées par une sonde calcique fluorescente puis exposées à 100 µM d'OAG, de 3-hydroxy-2,2-bis(hydroxyméthyl)propyl oléate, de mannide monooléate ou de N,N-diéthanololéamide. Les évolutions d'intensité de fluorescence, associées à des mouvements calciques intracellulaires, ont ensuite été enregistrées (voir Vachel *et al*., 2013). Les résultats obtenus indiquent que les 3-hydroxy-2,2-bis(hydroxyméthyl)propyl oléate et mannide monooléate exercent une influence très faible sur la vidange des stocks calciques cellulaires (en d'autres termes ils n'induisent pas de mobilisation calcique cellulaire) par rapport à l'OAG, et que ces composés présentent donc des risques très limités de toxicité cellulaire.

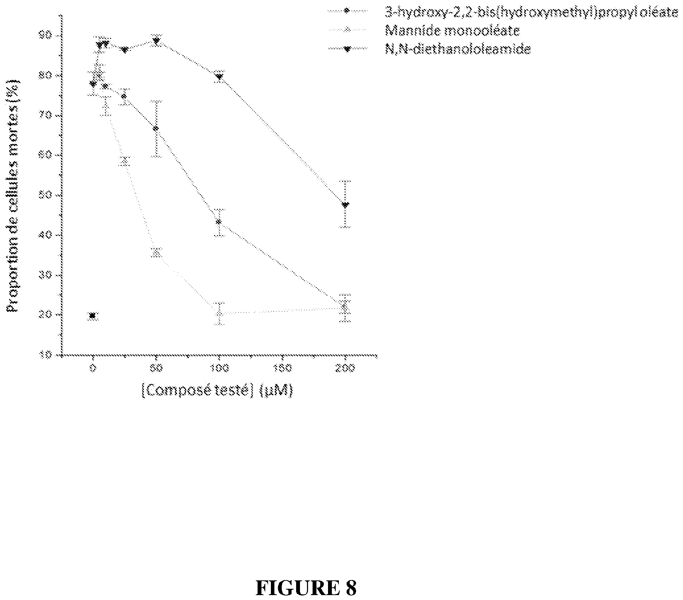

**Figure 8** **: Les 3-hydroxy-2,2-bis(hydroxyméthyl)propyl oléate, mannide monooléate et N,N-diéthanololéamide préviennent l'apoptose des cellules β-pancréatiques en présence d'acides gras saturés.**
   Des cellules β-pancréatiques BRIN-BD11 ont été cultivées en présence d'une source exogène d'acide gras saturé (acide palmitique, 200 µM), comme décrit par Dhayal & Morgan (2011) afin de générer des conditions de lipo-intoxication, avant l'addition de 3-hydroxy-2,2-bis(hydroxyméthyl)propyl oléate, mannide monooléate ou N,N-diéthanololéamide. En complément des données de la figure 6, ces résultats indiquent que les trois composés d'intérêt préviennent la mort des cellules β-pancréatiques induite par lipo-intoxication.

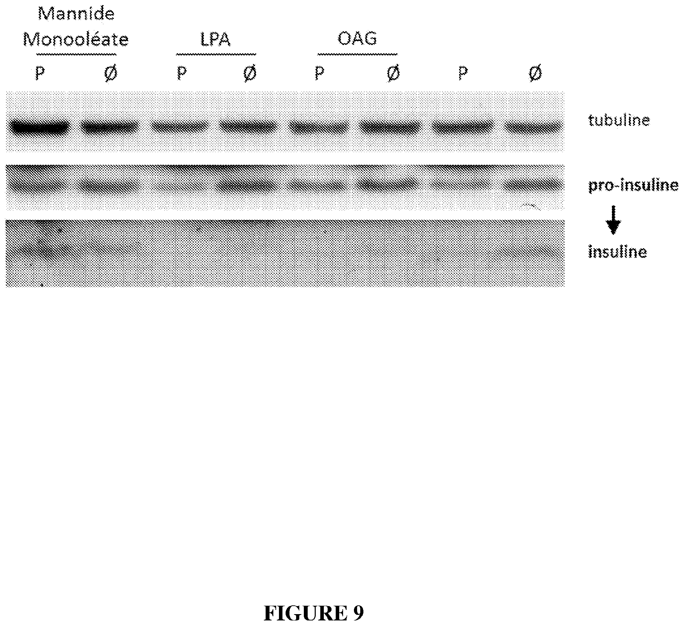

**Figure 9** **: Le mannide monooléate restaure la maturation de la pro-insuline dans des conditions de lipo-intoxication de cellules β-pancréatiques de mammifère.**
   Des cellules β-pancréatiques MIN6 ont été cultivées dans des conditions contrôle (Ø) ou, alternativement, en présence d'une source exogène d'acide palmitique à 400 µM (P), pendant 48 h, comme décrit par Boslem *et al*. (2011), afin de générer des conditions de lipo-intoxication. Lors des 24 dernières heures de culture, 200 µM d'OAG, de LPA ou de mannide monooléate ont, ou non, été ajoutés, comme mentionné. Dans ces conditions, les échantillons protéiques ont été soumis à un western blot et les résultats obtenus indiquent que seul le mannide monooléate restaure la maturation de la pro-insuline en insuline, dans des conditions de lipo-intoxication.

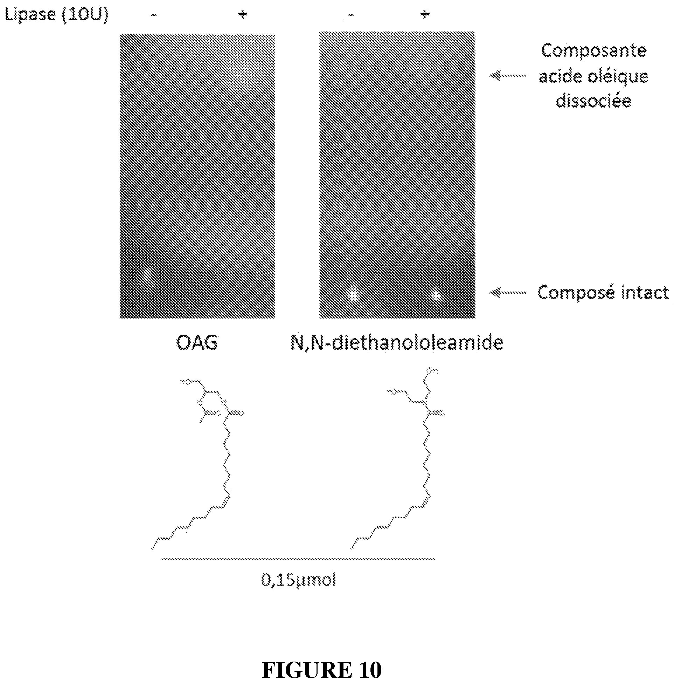

**Figure 10** **: Le N,N-diéthanololéamide résiste à l'activité hydrolytique de lipases.**
   15 µmol d'OAG et de N,N-diéthanololéamide ont été soumis (+), ou non (-), à une exposition à 10 U de lipases pendant 30 min à 37°C. Suite à l'incubation, les espèces lipidiques ont été extraites des échantillons avant d'être séparées par chromatographie sur couche mince. Les espèces moléculaires d'intérêt sont annotées et les résultats indiquent que, contrairement à l'OAG, le N,N-diéthanololéamide résiste à l'hydrolyse par des lipases.

### EXEMPLES

### A/ Souches de levures et lignées de cellules de mammifères

Les souches de levures *Saccharomyces cerevisiae* listées dans le tableau 1 sont utilisées pour les différents tests de restauration de croissance, pour la mise en évidence des toxicités, pour l'analyse du contenu en acides gras des phospholipides cellulaires ainsi que pour les tests de déclenchement de l'« unfolded protein response » (UPR).
L'état d'activation de l'UPR et l'induction de la mort cellulaire par lipo-intoxication ont également été analysés dans des lignées β pancréatiques de rat, BRIN-BD11.
Par ailleurs, les tests de mobilisation calcique ont été réalisés sur des cellules épithéliales humaines, CFBE, et les expériences de maturation de l'insuline ont été réalisées sur une lignée β pancréatique murine, MIN6.

**Tableau 1 : souches de levures utilisées**

| **Souche** | **Génotype** | **Origine** |
|---|---|---|
| *hem1Δ* | ***MATa** trp1 his3 ura3 leu2 hem1::LEU2* | FY1679α x FYHO4 |
| **QM** (H1246 W303) | ***MATα** are1::HIS3 are2::LEU2 dga1::KanMX4 lro1::TRP1 ADE2 met ura3* | ScanBi Ltd., Alnarp, Sweden |
| **WT** (G175 W303) | ***MATa** ADE2 MET his3 leu2 ura3 trp1* | ScanBi Ltd., Alnarp, Sweden |

### B/ Lipo-intoxication des levures hem1Δ

La souche portant la mutation *hem1*Δ est cultivée, en conditions aérobies, sous agitation et à 28°C, dans un milieu liquide YPG^{A} (YPG (extrait de levure 1 % (m/v), peptone 1 % (m/v) et glucose 2 % (m/v)) supplémenté avec de l'acide δ-aminolevulinique (ALA) à 80 µg/mL). La lipo-intoxication par des acides gras saturés (AGS) est provoquée par déplétion en acides gras insaturés (AGI) - dont la synthèse est dépendante de la présence d'hème (groupement prosthétique de l'enzyme Ole1p notamment) - par transfert en milieu YPG⁺ (YPG supplémenté d'ergostérol à 80 µg/mL pour compenser la déplétion en stérol obtenue dans cette condition). La lipo-intoxication peut être induite sur milieu solide YPG⁺ + agar 2 % (m/v) en transférant 3500 cellules (*hem1*Δ issues d'une préculture en YPG^{A})/cm² ou, alternativement, en milieu liquide en inoculant 2.10⁶ cellules/mL de YPG⁺. Classiquement, les effets de la lipo-intoxication aux AGS sont analysés 7h après le transfert en milieu YPG⁺. La capacité d'un composé à contrer les effets délétères d'une lipo-intoxication aux AGS est, quant à elle, évaluée successivement à l'ajout de ce composé sur (ou dans) le milieu de transfert YPG⁺, après l'ensemencement avec les cellules.

### C/ Lipo-intoxication de cellules β-pancréatiques de rat par l'acide palmitique

### 1) Préparation des réactifs lipidiques:

Les espèces lipidiques sont préparées dans de l'éthanol avant d'être complexées à de l'albumine de sérum bovin (BSA, initialement dépourvue d'acides gras) par une incubation de 1 heure à 37°C. Le stock d'acide palmitique est obtenu par l'ajout d'un volume d'éthanol avant que l'ensemble ne soit chauffé à 70°C pour homogénéisation. Les solutions d'OAG et de LPA sont préparées dans de l'éthanol 100% à température ambiante. Pour les incubations de cellules de mammifères, les concentrations finales de BSA et d'éthanol dans le milieu de culture sont respectivement maintenues à 1 et 0,5 % (m/v).

### 2) Tests de viabilité cellulaire:

La lignée de cellules β-pancréatiques (BRIN-BD11) de rat est cultivée dans du milieu RPMI-1640 complet, contenant du glucose à 11 mM et supplémenté a 10 % (v/v) de sérum de veau foetal (SVF), 2 mM de L-glutamine, 100 U/mL de pénicilline et 100 µg/mL de streptomycine. Pour chaque expérience, les cellules sont initialement ensemencées à une densité de 0,5×10⁵ cellules/mL dans des boites 6 puits pendant 24 heures. Par la suite, le milieu complet est remplacé par un équivalent dépourvu de SVF mais contenant les réactifs lipidiques d'intérêt, aux concentrations désirées, complexés à de la BSA. Dans le cas des conditions contrôles, des quantités identiques de BSA et d'éthanol sont alors utilisées. Au terme des incubations, l'ensemble des cellules (mortes et vivantes) est collecté et centrifugé à 300 g pendant 5 min. Le culot cellulaire est ensuite remis en suspension dans 200 µL de milieu puis l'ADN des cellules mortes (ayant perdu l'intégrité de leur membrane plasmique) est marqué à l'iodure de propidium (IP) en ajoutant 200 µL d'une solution d'IP à 20 µg/mL dans du tampon FACS (phosphate-buffered saline (PBS), 2 % (v/v) SVF, azoture de sodium 10 mM). Après une incubation de 10 min sur glace, les échantillons ainsi obtenus sont analysés par cytométrie en flux. Un Beckman Coulter EPICS XL MCL est utilisé pour la quantification, un canal FL3 sert à la détection des émissions de l'IP intercalé dans l'ADN et l'analyse est réalisée à l'aide du logiciel EXPO32 ADC (Applied Cytometry Systems, V 1.1 build 207).

### 3) Western blotting:

Les cellules BRIN-BD11 sont ensemencées à une densité de 0,5×10⁵ cellules/mL dans des flasques T25 pendant 24 heures. Comme indiqué précédemment, le milieu complet est alors remplacé par un équivalent dépourvu de SVF mais contenant les réactifs lipidiques d'intérêt. Après 6 heures d'incubation, l'extraction des protéines totales est réalisée à l'aide d'un tampon de lyse (Tris 20 mM, NaCl 150 mM, EDTA 1 mM et Triton-X 1 % (v/v)) contenant des inhibiteurs de protéases et de phosphatases. Ces protéines sont alors soumises à une électrophorèse en gel d'acrylamide 12 % NuPAGE® Novex® Bis-Tris Gels (Invitrogen) avant d'être transférées sur membrane de PVDF puis sondées à l'aide d'anticorps anti-phosphoeIF2α (Cell Signalling (New England Biolabs)) dilués au 1/1000^{ième}. Dans un second temps, les membranes sont décapées avec le tampon Re-Blot Plus-Strong (Millipore) avant d'être sondées une seconde fois avec des anticorps anti-eIF2α total (Cell Signalling (New England Biolabs) dilués au 1/1000^{ième}. L'analyse par densitométrie de l'abondance relative des formes phosphorylées ou non phosphorylées de la protéine eIF2α est effectuée avec le système Fluor-S Multi-imager analysis system combiné au logiciel Quantify One (Biorad UK Ltd).

### D/ Suivi de maturation de l'insuline (cf. Figure 9)

A la manière de ce qui est décrit précédemment pour la lipo-intoxication des cellules BRIN-BD11, la lignée MIN6 est cultivée dans un milieu DMEM-High Glucose (6 mM) complet, supplémenté à 10 % (v/v) de sérum de veau foetal (SVF), 15 mM d'HEPES, 100 U/mL de pénicilline et 100 µg/mL de streptomycine et la lipo-intoxication est induite par exposition à 400 µM d'acide palmitique, couplé à de la BSA (0,92% (w/v) final), pendant 48h, avec ou sans ajout de composé d'intérêt (voir Boslem *et al*., 2011). Les cellules sont ensuite collectées et un western blot est réalisé ainsi qu'indiqué précédemment, en utilisant des anticorps anti-insuline afin de suivre la maturation de la pro-insuline en insuline.

### E/ Test de mobilisation calcique (cf. Figure 7)

La lignée de cellules épithéliales humaines, CFBE, est cultivée sur boite à fond en verre dans un milieu MEM + GlutaMAX™-1 (aMEM ; Invitrogen) supplémenté de 10 % de sérum de veau foetal (SVF), de pénicilline à 100 IU/mL, de streptomycine à 100 µg/mL et de puromycine à 0,5 µg/mL. Les cellules sont initialement chargées avec 3 µM de sonde calcique fluorescente Fluo-4-acetoxymethyl ester (FluoProbes®), pendant 20 min à température ambiante. La mobilisation calcique est ensuite enregistrée par l'acquisition des évolutions d'intensité de fluorescence, pour une zone d'intérêt, grâce à un microscope inversé Zeiss Axio observer Z1, pour des séquences de stimulation laser de 250 ms, pendant 4 min. Les données collectées sont alors interprétées par utilisation du logiciel Carl Zeiss AxioVision Release 4.8.2 et du module d'acquisition physiologique associé. Les profils d'intensité sont finalement normalisés en divisant l'intensité à chaque pixel à un moment t (F) par l'intensité de fluorescence à ce pixel avant stimulation (Fo). Les images ((F-F₀)/F₀) ainsi obtenues permettent d'obtenir un profil d'intensité/de mobilisation calcique, sur l'ensemble de l'enregistrement (voir Vachel *et al*., 2013).

### F/ Restauration(s) de croissance

**1) Criblage de composés :** Suite à l'induction d'une lipo-intoxication aux AGS (pour des *hem1*Δ cultivées sur un milieu solide) des gouttes de 5 µL de solutions de différents composés à 10 mM dans du diméthylsulfoxide (DMSO) ou dans de l'éthanol (EtOH) sont déposées à la surface de l'agar. La capacité d'un composé à contrer l'arrêt de croissance cellulaire lipo-induit est estimée par l'apparition d'un halo de colonies de *hem1*Δ à l'emplacement du dépôt dudit composé après 3 jours de culture à 28°C (cf. Deguil *et al*., 2011).
**2) Cinétique de prolifération :** Conjointement à l'induction d'une lipo-intoxication aux AGS (pour des *hem1*Δ en milieu liquide) différents composés sont ajoutés aux cultures à une concentration initiale de 200 µM. Le suivi de prolifération est réalisé en mesurant la densité cellulaire par spectrométrie, à intervalles de temps réguliers (toutes les heures sur la durée de l'observation). A une longueur d'onde de 600 nm, une unité de densité optique (DO_{600 nm}) correspond à 2.10⁷ cellules/mL.

### G/ Test de toxicité

Parallèlement, les souches sauvages (WT) et QM sont cultivées, en condition aérobie, sous agitation et à 28°C, dans un milieu liquide YPG avant d'ensemencer 3500 cellules par cm² de YPG + agar 2 % (m/v). Suite à ce transfert sur milieu solide, des gouttes de 1 µL de solutions de différents composés à 1, 10 et 100 mM dans du DMSO ou de l'EtOH sont déposées à la surface de l'agar. Séparément, des dépôts de DMSO et d'EtOH sont également réalisés afin d'évaluer la toxicité intrinsèque de ces deux solvants. Après 3 jours de culture à 28°C, la toxicité des composés testés est évaluée en comparant les diamètres des halos d'inhibition de croissance obtenus pour les dépôts de solvants bruts à ceux des dépôts des différentes concentrations de composés testés. Contrairement à la souche WT, la souche QM est incapable de tamponner un excès d'acide oléique exogène sous forme de lipides neutres (triglycérides (TG) ou esters de stérols (ES)) dans des gouttelettes lipidiques. Ainsi, dans le cas d'une absence de toxicité vis-à-vis de la souche WT, l'observation d'une toxicité d'un composé vis-à-vis de la souche QM indique que ce composé est perçu comme une source d'acide gras libre par les levures.

### H/ Extraction de lipides totaux

La souche *hem1*Δ est cultivée en milieu liquide YPG^{A}, YPG⁺ ou YPG⁺ + 200 µM de composé à tester, en aérobie, sous agitation et à 28°C pendant 7 h, à partir d'une concentration cellulaire initiale de 2.10⁶ cellules/mL. A l'issue de la culture, 10⁸ cellules sont collectées afin de réaliser l'extraction des lipides totaux. Après avoir mis les cellules en suspension dans 1 mL d'eau distillée à 4°C, 500 µM de billes de verre (Ø 0,6 mm) sont ajoutés et l'ensemble subit alors 3 séquences de 20 sec à 5000 tours/min dans un agitateur (les tubes sont maintenus sur glace entre chacune des 3 séquences). Le lysat cellulaire alors obtenu, complété de l'eau de rinçage des billes (1 mL), est ensuite transféré dans un tube en verre de 40 mL (Corex™) avant de réaliser l'extraction des lipides en utilisant un ratio méthanol:chloroforme 2:1 (v/v). Initialement, 6 mL de méthanol sont ajoutés et l'ensemble est vortexé pendant 30 sec puis incubé pendant 15 min à 65°C. Une fois le mélange refroidi à température ambiante, 3 mL de chloroforme sont ajoutés puis l'ensemble est à nouveau vortexé pendant 30 sec avant de laisser l'extraction se dérouler pendant 16 h. Ultérieurement, l'échantillon est centrifugé pendant 12 min à 10000 g avant de transférer le surnageant dans un nouveau tube Corex™. Après l'ajout de 2 mL de chloroforme puis de 4 mL d'eau distillée, l'ensemble est vortexé pendant 30 sec puis centrifugé pendant 8 min à 3000 g. Après élimination de la phase supérieure résultante, la phase organique inférieure est collectée dans un tube à hémolyse en verre. Finalement, le solvant est évaporé sous flux d'azote à 80°C pour obtenir les échantillons de lipides cellulaires totaux.

### I/ Purification de phospholipides et analyse par spectrométrie de masse

Les échantillons de lipides cellulaires totaux sont remis en suspension dans 1 mL de dichlorométhane en étant vortexés pendant 30 secondes. L'ensemble est déposé sur une colonne de silice (BOND ELUT-SI, 100 mg 1 mL) pré-conditionnée avec 3 mL de méthanol puis 2 mL de dichlorométhane successivement. La fraction retenue par la colonne est ensuite lavée avec 2 mL de dichlorométhane puis 3 mL d'acétone successivement. Finalement, 2 mL d'un mélange de chloroforme/méthanol/eau 50:45:5 (v/v/v) sont déposés sur la colonne et les phospholipides ainsi élués sont collectés dans un tube à hémolyse en verre. Le solvant est évaporé sous azote à 80°C pour obtenir les échantillons de phospholipides cellulaires.
Une fois remis en suspension dans 100 µL de mélange Mix⁻ (isopropanol/acétonitrile/eau 2:1:1 (v/v/v) + triéthylamine 1 % (v/v)) ou de mélange Mix⁺ (isopropanol/acétonitrile/eau 2:1:1 (v/v/v) + acide formique 1 % (v/v)), les échantillons sont analysés par spectrométrie de masse (ElectroSpray Ionization-Mass pectrometry (ESI-MS)), en mode négatif ou positif respectivement, et les résultats obtenus servent à analyser le contenu en acide gras des différentes espèces de phospholipide.

### J/ Test de déclenchement de l'UPR

La souche *hem1*Δ transformée par le plasmide pPW344 [2µ URA3 4×UPRE-*LacZ* (Patil *et al*., 2004)] est cultivée en milieu liquide YPG^{A}, YPG ou YPG + 200 µM de composé à tester, en condition aérobie, sous agitation et à 28°C pendant 7 h, à partir d'une concentration cellulaire initiale de 2.10⁶ cellules/mL. A l'issue de la culture, 10⁸ cellules sont collectées afin de quantifier l'activité beta-galactosidase (β-gal) résultante de l'expression du transgène *LacZ* (dans le cas d'une activation de l'UPR). Dans un premier temps, les cellules sont remises en suspension dans 1,5 mL de tampon Z (Na₂HPO₄ à 60 mM, NaH₂PO₄ à 40 mM, KCl à 10 mM, MgSO₄ à 1 mM et β-mercaptoéthanol à 50 mM ; solution à pH 7) puis 1/15^{ième} de cette suspension est utilisé pour réaliser une mesure de DO_{600 nm}. Dans un second temps, la suspension est complétée avec 100 µL de sodium dodecyl sulfate (SDS) 0,1 % (v/v) et 200 µL de chloroforme puis vortexée en deux séquences de 30 sec successives. Après décantation, 400 µL (volume V) de la solution ainsi obtenue sont transférés dans un tube à hémolyse en verre puis complétés de 600 µL de tampon Z. 200 µL de substrat ortho-nitrophényl-β-galactoside (ONPG), à 4 mg/mL dans du tampon Z, sont alors ajoutés avant que l'ensemble soit homogénéisé au vortex puis incubé au bain marie à 30°C pour initier la réaction. Lorsque l'ensemble présente une légère teinte jaune, la réaction est interrompue (au temps t), à température ambiante, par ajout de 500 µL de Na₂CO₃ à 1M. Finalement, après avoir centrifugé les échantillons pendant 5 min à 800 g puis collecté les surnageants dans de nouveaux tubes à hémolyse en verre, les produits de la réaction (o-nitrophénol) ainsi que les débris cellulaires sont dosés par spectrométrie aux longueurs d'onde 420 et 550 nm respectivement. Pour chaque échantillon, l'activité β-gal (U) est calculée grâce à la formule U=(1000×[DO₄₂₀ₙₘ-(1,75×DO₅₅₀ₙₘ)])/(t×V×DO₆₀₀ₙₘ), exprimée en unité arbitraire.

### REFERENCES

- Alkhateeb H, Chabowski A, Glatz JFC, Luiken JFP, Bonen A (2007) Two phases of palmitate-induced insulin résistance in skeletal muscle: impaired GLUT4 translocation is followed by a reduced GLUT4 intrinsic activity. American Journal of Physiology - Endocrinology And Metabolism 293: E783-E793
- Bigay J, Casella JF, Drin G, Mesmin B, Antonny B. ArfGAP1 responds to membrane curvature through the folding of a lipid packing sensor motif. EMBO J. 2005 Jul 6;24(13):2244-53.
- Boslem E, MacIntosh G, Preston AM, Bartley C, Busch AK, Fuller M, Laybutt DR, Meikle PJ, Biden TJ. A lipidomic screen of palmitate-treated MIN6 β-cells links sphingolipid metabolites with endoplasmic reticulum (ER) stress and impaired protein trafficking. Biochem J. 2011 Apr 1;435(1):267-76.
- Butler AE, Janson J, Bonner-Weir S, Ritzel R, Rizza RA, Butler PC. (2003) Î2-Cell Deficit and Increased Î2-Cell Apoptosis in Humans With Type 2 Diabetes. Vol. 52, pp. 102-110.
- Cnop M, Hannaert JC, Hoorens A, Eizirik DL, Pipeleers DG (2001) Inverse Relationship Between Cytotoxicity of Free Fatty Acids in Pancreatic Islet Cells and Cellular Triglyceride Accumulation. Diabetes 50: 1771-1777.
- Cunha DA, Hekerman P, Ladriere L, Bazarra-Castro A, Ortis F, Wakeham MC, Moore F, Rasschaert J, Cardozo AK, Bellomo E, Overbergh L, Mathieu C, Lupi R, Hai T, Herchuelz A, Marchetti P, Rutter GA, Eizirik DL, Cnop M. (2008) Initiation and execution of lipotoxic ER stress in pancreatic {beta}-cells. Vol. 121, pp. 2308-2318.
- Deguil J, Pineau L, Rowland Snyder EC, Dupont S, Beney L, Gil A, Frapper G, Ferreira T (2011) Modulation of Lipid-Induced ER Stress by Fatty Acid Shape. Trafic 12: 349-362
- Dhayal S, Morgan NG (2011) Structure-activity relationships influencing lipid-induced changes in eIF2alpha phosphorylation and cell viability in BRIN-BD11 cells. FEBS Lett 585: 2243-2248
- Diakogiannaki E, Morgan NG. (2008) Differential régulation of the ER stress response by long-chain fatty acids in the pancreatic β-cell. Vol. 036, pp. 959-962.
- Diakogiannaki E, Welters HJ, Morgan NG. (2008) Differential régulation of the endoplasmic reticulum stress response in pancreatic {beta}-cells exposed to long-chain saturated and monounsaturated fatty acids. Vol. 197, pp. 553-563.
- Egnatchik RA, Leamy AK, Jacobson DA, Shiota M, Young JD. ER calcium release promotes mitochondrial dysfunction and hepatic cell lipotoxicity in response to palmitate overload. Mol Metab. 2014 May 22; 3(5):544-53.
- Guo W, Wong S, Xie W, Lei T, Luo Z. (2007) Palmitate modulates intracellular signaling, induces endoplasmic reticulum stress, and causes apoptosis in mouse 3T3-L1 and rat primary preadipocytes. Vol. 293, pp. E576-586.
- Iwasaki Y, Saito O, Tanabe M, Inayoshi K, Kobata K, Uno S, Morita A, Watanabe T. Monoacylglycerols activate capsaicin receptor, TRPV1. Lipids. 2008 Jun;43(6):471-83.
- Kato T, Shimano H, Yamamoto T, Ishikawa M, Kumadaki S, Matsuzaka T, Nakagawa Y, Yahagi N, Nakakuki M, Hasty AH, Takeuchi Y, Kobayashi K, Takahashi A, Yatoh S, Suzuki H, Sone H, Yamada N. (2008) Palmitate Impairs and Eicosapentaenoate Restores Insulin Secretion Through Regulation of SREBP-1c in Pancreatic Islets. Vol. 57, pp. 2382-2392.
- Katsoulieris E, Mabley JG, Samai M, Green IC, Chatterjee PK (2009) [alpha]-Linolenic acid protects renal cells against palmitic acid lipotoxicity via inhibition of endoplasmic reticulum stress. European Journal of Pharmacology 623: 107-112
- Kincaid MM, Cooper AA (2007) ERADicate ER Stress or Die Trying. Antioxid Redox Signal
- Kohlwein SD, Petschnigg J (2007) Lipid-induced cell dysfunction and cell death: lessons from yeast. Current hypertension reports 9: 455-461
- Laybutt DR, Preston AM, Akerfeldt MC, Kench JG, Busch AK, Biankin AV, Biden TJ (2007) Endoplasmic reticulum stress contributes to beta cell apoptosis in type 2 diabetes. Diabetologia 50: 752-763
- Listenberger LL, Han X, Lewis SE, Cases S, Farese RV, et al. (2003) Triglyceride accumulation protects against fatty acid-induced lipotoxicity. PNAS 100: 3077-3082
- Martin AC, Cooper DM. Capacitative and 1-oleyl-2-acetyl-sn-glycerol-activated Ca(2+) entry distinguished using adenylyl cyclase type 8. Mol Pharmacol. 2006 Aug;70(2):769-77
- Patil CK, Li H, Walter P. Gcn4p and novel upstream activating sequences regulate targets of the unfolded protein response. PLoS Biol. 2004 Aug;2(8):E246
- Payet LA, Pineau L, Snyder EC, Colas J, Moussa A, Vannier B, Bigay J, Clarhaut J, Becq F, Berjeaud JM, Vandebrouck C, Ferreira T. Saturated fatty acids alter the late secretory pathway by modulating membrane properties. Traffic. 2013 Sep 6.
- Petschnigg J, Moe OW, Stagljar I (2011) Using yeast as a model to study membrane proteins. Curent opinion in nephrology and hypertension 20: 425-432
- Petschnigg J, Wolinski H, Kolb D, Zellnig Gn, Kurat CF, Natter K, Kohlwein SD (2009) Good Fat, Essential Cellular Requirements for Triacylglycerol Synthesis to Maintain Membrane Homeostasis in Yeast. J Biol Chem 284: 30981-30993
- Pineau L, Bonifait L, Berjeaud J-M, Alimardani-Theuil P, Berges T, Ferreira T (2008) A Lipid-mediated Quality Control Process in the Golgi Apparatus in Yeast. Mol Biol Cell 19: 807-821
- Pineau L, Colas J, Dupont S, Beney L, Fleurat-Lessard P, Berjeaud JM, Berges T, Ferreira T (2009) Lipid-Induced ER Stress: Synergistic Effects of Sterols and Saturated Fatty Acids. Traffic
- Pineau L, Ferreira T (2010) Lipid-induced ER stress in yeast and P cells: parallel trails to a common fate. FEMS Yeast Research
- Poon PP, Nothwehr SF, Singer RA, Johnston GC. The Gcs1 and Age2 ArfGAP proteins provide overlapping essential function for transport from the yeast trans-Golgi network. J Cell Biol. 2001 Dec 24;155(7):1239-50
- Robinson M, Poon PP, Schindler C, Murray LE, Kama R, Gabriely G, Singer RA, Spang A, Johnston GC, Gerst JE. The Gcs1 Arf-GAP mediates Snc1,2 v-SNARE retrieval to the Golgi in yeast. Mol Biol Cell. 2006 Apr;17(4):1845-58
- Schneider MF, Marsh D, Jahn W, Kloesgen B, Heimburg T. Network formation of lipid membranes: triggering structural transitions by chain melting. Proc Natl Acad Sci USA. 1999 Dec 7;96(25):14312-7
- Stein DT, Stevenson BE, Chester MW, Basit M, Daniels MB, Turley SD, McGarry JD (1997) The insulinotropic potency of fatty acids is influenced profoundly by their chain length and degree of saturation. The Journal of Clinical Investigation 100: 398-403
- Stutzmann GE, Mattson MP. Endoplasmic reticulum Ca(2+) handling in excitable cells in health and disease. Pharmacol Rev. 2011 Sep;63(3):700-27
- Wei Y, Wang D, Topczewski F, Pagliassotti MJ. (2006) Saturated fatty acids induce endoplasmic reticulum stress and apoptosis independently of ceramide in liver cells. Vol. 291, pp. E275-281.
- Zhang K, Kaufman RJ. (2006) The unfolded protein response: A stress signaling pathway critical for health and disease. Vol. 66, pp. S102-109.
- Vachel L, Norez C, Becq F, Vandebrouck C. Effect of VX-770 (ivacaftor) and OAG on Ca2+ influx and CFTR activity in G551D and F508del-CFTR expressing cells. J. Cyst. Fibros. 2013 Dec;12(6):584-91.

## Revendications

1. Composé sélectionné parmi le mannide monoléate, le 3-hydroxy-2,2-bis(hydroxymethyl)propyl oléate et le N,N-diethanololeamide pour une utilisation pour prévenir ou traiter le syndrome métabolique, le diabète de type II ou la stéatose hépatique chez un sujet.

2. Composé selon la revendication 1, pour une utilisation pour prévenir ou traiter le syndrome métabolique, typiquement au moins un symptôme du syndrome métabolique sélectionné parmi insulino-résistance, hyperglycémie, hypercholestérolémie, hypertriglycéridémie, hypertension, insuffisance cardiaque et stéatose hépatique.

3. Composé selon la revendication 1 pour une utilisation pour prévenir ou traiter le diabète de type 2 en combinaison avec un composé distinct utilisé classiquement dans la prévention ou le traitement du diabète de type 2, ledit composé distinct étant de préférence choisi parmi biguanide, glitazone, sulfamide hypoglycémiant, glinide, inhibiteur de DPP4, incrétino-mimétique et inhibiteur d'α-Glucosidase.

4. Composé pour utilisation selon l'une quelconque des revendications 1 à 3, où ledit sujet est un animal, typiquement un mammifère, de préférence un être humain.

5. Composition comprenant un composé sélectionné parmi le mannide monoléate, le 3-hydroxy-2,2-bis(hydroxymethyl)propyl oléate et le N,N-diethanololeamide pour une utilisation pour prévenir ou traiter le syndrome métabolique, le diabète de type 2 et/ou la stéatose hépatique chez un sujet.

6. Composition pour utilisation selon la revendication 5, ladite composition étant une choisie parmi une composition pharmaceutique, un alicament et un supplément alimentaire.

## Patentansprüche

1. Verbindung, ausgewählt aus Mannidmonooleat, 3-Hydroxy-2,2-bis(hydroxymethyl)propyloleat und N,N-Diethanololeamid, für eine Verwendung zur Prävention oder Behandlung des metabolischen Syndroms, des Diabetes Typ II oder der Steatosis hepatis bei einem Lebewesen.

2. Verbindung gemäß Anspruch 1 für eine Verwendung zur Prävention oder Behandlung des metabolischen Syndroms, typischerweise wenigstens eines Symptoms des metabolischen Syndroms, ausgewählt aus Insulinresistenz, Hyperglykämie, Hypercholesterolämie, Hypertriglyceridämie, Hypertonie, Herzinsuffizienz und Steatosis hepatis.

3. Verbindung gemäß Anspruch 1 für eine Verwendung zur Prävention oder Behandlung des Diabetes Typ II in Kombination mit einer separaten Verbindung, die herkömmlicherweise in der Prävention oder Behandlung des Diabetes Typ II eingesetzt wird, wobei besagte separate Verbindung bevorzugt aus Biguanid, Glitazon, hypoglykämischem Sulfamid, Glinid, DPP4-Hemmer, Inkretinmimetikum und α-Glucosidase-Hemmer ausgewählt ist.

4. Verbindung zur Verwendung gemäß einem der Ansprüche 1 bis 3, wobei besagtes Lebewesen ein Tier, typischerweise ein Säugetier, bevorzugt ein Mensch ist.

5. Zusammensetzung, umfassend eine Verbindung, ausgewählt aus Mannidmonooleat, 3-Hydroxy-2,2-bis(hydroxymethyl)propyloleat und N,N-Diethanololeamid, für eine Verwendung zur Prävention oder Behandlung des metabolischen Syndroms, des Diabetes Typ II und/oder der Steatosis hepatis bei einem Lebewesen.

6. Zusammensetzung zur Verwendung gemäß Anspruch 5, wobei besagte Zusammensetzung aus einer pharmazeutischen Zusammensetzung, einem funktionellen Lebensmittel und einem Nahrungsergänzungsmittel ausgewählt ist.

## Claims

1. Compound selected from mannide monooleate, 3-hydroxy-2,2-bis(hydroxymethyl)propyl oleate and N,N-diethanololeamide for use in preventing or treating metabolic syndrome, type 2 diabetes mellitus or hepatic steatosis in a subject.

2. Compound according to claim 1, for use in preventing or treating metabolic syndrome, typically at least one symptom of metabolic syndrome selected from insulin resistance, hyperglycemia, hypercholesterolemia, hypertriglyceridemia, hypertension, heart failure and hepatic steatosis.

3. Compound according to claim 1, for use in preventing or treating type 2 diabetes mellitus in combination with a distinct compound classically used in the prevention or treatment of type 2 diabetes mellitus, said distinct compound preferably being chosen from biguanide, glitazone, sulfonamide-based hypoglycemic, glinide, DPP4 inhibitor, incretin mimetic and α-glucosidase inhibitor.

4. Compound for use according to any one of claims 1 to 3, where said subject is an animal, typically a mammal, preferably a human being.

5. Composition comprising a compound selected from mannide monooleate, 3-hydroxy-2,2-bis(hydroxymethyl)propyl oleate and N,N-diethanololeamide for use in preventing or treating metabolic syndrome, type 2 diabetes mellitus and/or hepatic steatosis in a subject.

6. Composition for use according to claim 5, said composition being chosen from a pharmaceutical composition, a functional food and a food supplement.
